# EUROPÄISCHE PATENTSCHRIFT

(11) **EP 1 320 571 B1**
(45) Veröffentlichungstag und Bekanntmachung des Hinweises auf die Patenterteilung: **21.11.2007**
(21) Anmeldenummer: 01962995.5
(22) Anmeldetag: 03.09.2001
(51) Int. Cl.: C09B 23/00, C07D 471/10, C07D 491/107, G01N 33/50

(54) **3-SPIRO-CYANIN FLUOROCHROME UND DEREN VERWENDUNG IN BIOASSAYS**
3-SPIRO-CYANIN FLUOROCHROMES AND THEIR USE IN BIOASSAYS
FLUOROCHROMES A 3-SPIROCYANINES ET LEUR UTILISATION DANS DES ESSAIS BIOLOGIQUES

(30) Priorität: 19.09.2000 DE 10046215
(43) Veröffentlichungstag der Anmeldung: 25.06.2003
(73) Patentinhaber: ICB Institut für Chemo- und Biosensorik GmbH, 48149 Münster (DE)
(72) Erfinder: HAALCK, Lutz, 48149 Münster (DE); GEDIG, Erk, 48149 Münster (DE); HÖLPERT, Hans-Jürgen, 48157 Münster (DE); PODSADLOWSKI, Viola, 48143 Münster (DE)
(74) Vertreter: Pfenning, Meinig & Partner GbR
(86) Internationale Anmeldenummer: PCT/EP2001/010130
(87) Internationale Veröffentlichungsnummer: WO 2002/026890

(56) Entgegenhaltungen:
- EP-A- 0 710 668
- WO-A-93/06482
- WO-A-98/30992
- WO-A-98/58942
- DE-A- 3 912 046
- DE-A- 19 940 394
- MUJUMDAR R B ET AL: "CYANINE DYE LABELING REAGENTS: SULFOINDOCYANINE SUCCINIMIDYL ESTERS" BIOCONJUGATE CHEMISTRY, AMERICAN CHEMICAL SOCIETY, WASHINGTON, US, Bd. 4, Nr. 2, 1. März 1993 (1993-03-01), Seiten 105-111, XP000654181 ISSN: 1043-1802
- RANDOLPH J B ET AL: "STABILITY, SPECIFICITY AND FLUORESCENCE BRIGHTNESS OF MULTIPLY-LABELED FLUORESCENT DNA PROBES" NUCLEIC ACIDS RESEARCH, OXFORD UNIVERSITY PRESS, SURREY, GB, Bd. 25, Nr. 14, 15. Juli 1997 (1997-07-15), Seiten 2923-2929, XP002074426 ISSN: 0305-1048
- PATENT ABSTRACTS OF JAPAN vol. 1998, no. 12, 31. Oktober 1998 (1998-10-31) & JP 10 195319 A (MITSUBISHI PAPER MILLS LTD), 28. Juli 1998 (1998-07-28)
- RODRIGUEZ,J.G. ET AL.: "Synthesis of 2'-Alkylspiro[2-X-cyclohexane-1,3'-3'H-ind ole] (X = H;X = CH3) by an Unexpected Reaction between an Organomagnesium Halide and 2'-Methylspiro[2-X-cyclohexane-1,3'-3'H-in dole]. X-ray Structure of a Fluorescent Dimeric Compound" J.ORG.CHEM., Bd. 63, Nr. 13, 1998, Seiten 4332-4337, XP002185371 WASHINGTON

## Beschreibung

Die Erfindung betrifft sterisch abgeschirmte, stabilisierte Fluoreszenzfarbstoffe auf der Basis symmetrischer oder unsymmetrischer 3-Spirocyanine sowie deren Verwendung als Markermoleküle in analytischen Verfahren.

Fluoreszenzfarbstoffe werden in zunehmenden Maße zur Markierung von Enzymen, Antikörpern und Nukleinsäuren zum Einsatz u.a. in Immunoassays, der Fluoreszenzmikroskopie und zur Sequenzierung eingesetzt. Traditionell werden Fluorochrome eingesetzt, die im UV oder im sichtbaren Bereich angeregt werden. Ihre Anregungs- und Emissionsspektren überlappen jedoch mit der natürlichen Serumfluoreszenz, weshalb sie für die Analytik natürlicher Proben (z.B. Blut- und Serumsproben, Zellen) nur bedingt geeignet sind. Seit einigen Jahren werden daher zunehmend Fluorochrome mit Anregungswellenlängen im nahen infraroten Bereich des Lichtspektrums verwendet, die eine Messung biologischer Proben mit geringer Hintergrundfluoreszenz bei gleichzeitig gesteigerter Sensitivität ermöglichen.

Durch neueste wissenschaftliche Entwicklungen im Bereich der Proteomics und Genomics kommt es zu einem exponentiell wachsenden Bedarf an Biochips (Protein-, DNA-Arrays), die sowohl die spezifische Detektion von Proteinen als auch DNA, bzw. Fragmenten ermöglichen. In den meisten Fällen kommen hier ebenfalls Fluoreszenzfarbstoffe zum Einsatz.

In der US 5,268,486 wird erstmals der Einsatz reaktiver und wasserlöslicher NIR-Fluorochrome zur Kopplung an Biomoleküle beschrieben. Dazu werden chemische Gruppen, vorzugsweise als N-Substituent, eingeführt, die zur Kopplung mit reaktiven Aminosäureseitengruppen, z.B. Amino-, Thio-, Carbonyl-, oder Hydroxyfunktionen geeignet sind. Dazu gehören u.a. Isothiocyanante, Thiocyanante, Hydrazine, Hydroxysuccinimidester, Disulfide usw. Dieses Prinzip wurde in jüngster Zeit erweitert und umfaßt neben symetrischen Cyanomethinfarbstoffen (2,2'-Indocyaninen) auch die strukturell verwandten Merocyanine und Styrylfarbstoffe.

Aus der DE-A-199 40 394 sind 4,4'Cyaninfarbstoffe bekannt, die im Gegensatz zur vorliegenden Erfindung auf Chinolin-Kopfgruppen und nicht auf Indol-Kopfgruppen beruhen.

Die WO-A-98/30992 offenbart Cyaninfarbstoffe mit Sulfoalkyl-Substituenten am Stickstoffindol, die zusätzlich über eine reaktive Gruppe zur kovalenten Bindung an Biomoleküle verfügen. Die Indol-Kopfgruppen verfügen über (C(CH₃)₂ -Gruppen an Position 3.

In der EP-A-0710 668 werden Cyaninfarbstoffe mit einer Indol- und einer Chinolin-Kopfgruppe beschrieben. Die Position 3 am Indol offenbart O, Se, S, N-alkyl und C(CH₃)ₙ.

Die DE-A-39 120 46 beschreibt wasserlösliche Cyanine, die gleichzeitig über eine reaktive Gruppe zur kovalenten Kopplung verfügen. Im Gegensatz zur vorliegenden Erfindung werden Indol-Kopfgruppen verwendet, die in 3-Position (X und Y) O, S oder C(CH₃)₂ besitzen.

Aus Bioconj. Chem., 4, 105, 1993 sind ebenfalls wasserlösliche Cyanine bekannt. Die offenbarten Indolkopfgruppen verfügen jeweils über CH₃-Gruppen in Position 3.

In Nucl. Acids Res., 25, 2923 (1997) werden Cyaninfarbstoffe beschrieben, die neben Arylsulfonsäuren zur Wasserlöslichkeit eine reaktive Gruppe (hier ein Succinimidylester, NHS) zur Kopplung an Nukleinsäuren verfügen. Die Indolkopfgruppen verfügen jeweils über CH₃-Gruppen in Position 3.

In der WO-A-98/58942 werden nicht-sulfonierte Cyaninfarbstoffe zur Kopplung an Nukleotide/Nucleoside beschrieben. Die Position 3 der Indolkopfgruppe, hier als X und Y beschrieben, ist definiert als O, S, C(R⁶)₂, N(R⁶), wobei R⁶ bevorzugt CH₃ oder eine kurzkettige Alkylgruppe ist. Der Farbstoff ist kovalent an Nukleotide/Nucleoside gebunden und verfügt über keine reaktive Gruppe. Die Druckschrift offenbart keine Spirocyaninfarbstoffe.

Die WO-A-93/06 482 beschreibt Dimere unsymmetrischer Cyaninfarbstoffe. Die Indolgruppen der beteiligten Farbstoffe verfügen an der Position 3 (hier X und Z) über O, S, N-R₃, bzw. N-R₄, wobei R₃ und R₄ Wasserstoff oder eine Alkylgruppe bis zu 6 C-Atomen darstellt. Der Cyaninfarbstoff wird mittels ionischer Wechselwirkungen an DNA gebunden.

Die JP-A-10 195 319, JP-A-10 219 124 und J. Org. Chem. 63, 4332 (1998) offenbaren Cyaninfarbstoffe mit einem Spiranring in Position 3 der Indole. Darüber hinaus sind die Farbstoffe jedoch weder mit zusätzlichen wasserlöslichen Gruppen noch mit einer reaktiven Gruppen zur kovalenten Kopplung ausgestattet.

Die WO-A-97/13 490, die WO-A-98/47 538 und die WO-A-98/22 146 beschreiben ebenfalls wasserlösliche Cyaninfarbstoffe. Die verwendeten Indol-Kopfgruppen offenbaren in Position 3 (X und Y) O, S, CH=CH oder ein Fragment C(CH₂-R¹³)CH₂-R¹⁴, wobei die Reste R¹³ und R¹⁴ einen 5 oder 6-gliedrigen Ring (ein Spiran) ausbilden können. Im Gegensatz zur vorliegenden Erfindung verfügen die offenbarten Farbstoffe jedoch über keine reaktiven Gruppen zur kovalenten Kopplung.

Alle Farbstoffe dieses Typs (Rhodamine, Squaraine und Cyanine) zeigen ein charakteristisches Verhalten in wässriger Lösung. Hier kommt es aufgrund der planaren Anordnung der Chromophore bei höheren Kopplungsgraden (F/P > 5)zur Aggregation oder Dimerenbildung. Diese äußert sich durch strahlungslose intermolekulare Übergänge in einer drastischen Reduktion der resultierenden Fluoreszenz. Dieses Phänomen tritt sowohl in Lösung, z.B. in Gegenwart von hohen Salzgehalten, als auch bei Protein gebundenen Fluorophoren auf. Besonders empfindlich sind Farbstoffe ohne zusätzliche hydrophile Gruppen, aber auch aminosubstituierte Cyaninfarbstoffe.

Durch die im Stand der Technik beschriebenen Farbstoffe konnte dieses Problem durch die Einführung von Arylsulfonsäuregruppen zur Erhöhung der Wasserlöslichkeit reduziert, jedoch nicht beseitigt werden. Die Quantenausbeute in wässriger Lösung ist jedoch deutlich geringer als in organischen Lösungsmitteln.

Zum Markieren von Oligunukleotiden oder DNA/RNA wird eine vielzahl von Wechselwirkungen ausgenutzt. Neben den klassischen Intercalationsfarbstoffen, wie Ethidiumbromid kommen sowohl positive geladenen Farbstoffe zur spezifischen ionischen Bindung an die Phosphatgruppen der Nukleotide (US 5,410,030) als auch diverse kovalente Bindungen der Farbstoffe an modifizierte Purin/Pyrimidinbasen (US 6,027,709) oder an die Phosphatgruppen der Nukleotide (US 5,986,086) in Frage. Diese Entwicklungen spiegeln den in den letzten Jahren sprunghaft gestiegenen Bedarf an geeigneten Fluoreszenzfarbstoffen für die DNA-Markierung und Sequenzierung wider.

Ausgehend hiervon ist es die Aufgabe der vorliegenden Erfindung Fluorochrome vorzuschlagen, die eine hohe Quantenausbeute und gleichzeitig geringere Tendenz zur Aggregation aufweisen. Zusätzlich sollen diese Fluorochrome eine möglichst große Vielfalt hinsichtlich der Bindungsmöglichkeiten an diverse Zielmoleküle aufweisen, um so weite Anwendungsgebiete abzudecken.

Die Aufgabe wird durch die kennzeichnenden Merkmale des Patentanspruches 1 gelöst. Die weiteren abhängigen Ansprüche zeigen vorteilhafte Weiterbildungen auf. Die Verwendung der erfindungsgemäßen Fluorochrome wird gemäß den Ansprüchen 21 bis 24 beschrieben.

Erfindungsgemäß werden somit Fluorochrome vorgeschlagen, die auf einer Grundstruktur von symmetrischen oder unsymmetrischen Cyaninfarbstoffen mit mindestens einem sterisch anspruchsvollen Spiro-Substituenten in 3-Position der Indolkopfgruppe beruhen. Diese Fluorochrome werden im Weiteren auch als Spironine bezeichnet. Die erfindungsgemäßen Fluorochrome weisen dabei folgendes Grundgerüst auf:
Fluorochrom der allgemeinen Formel I 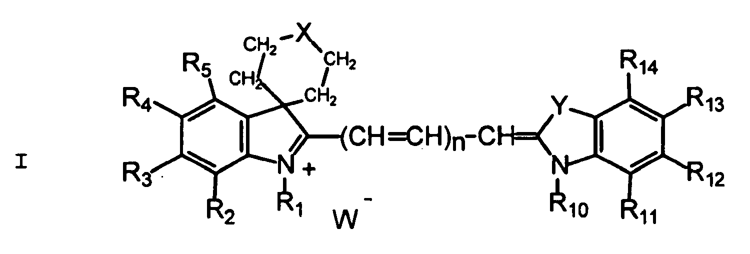 mit X = 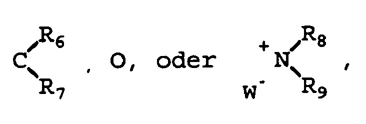 und Y ist, O, NR₁₇, 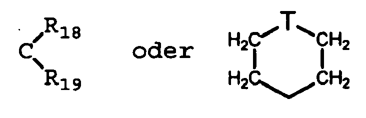 mit T gleich 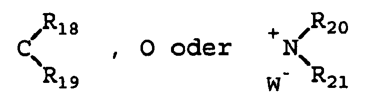 mit n = 0, 1, 2 oder 3 und
   W⁻ = Gegenion, vorzugsweise Halogenid, Perchlorat oder Tosylat,
wobei mindestens einer der Reste R₁ bis R₂₁ ausgewählt ist aus
einer reaktiven Gruppe zur kovalenten Ankopplung eines Zielmoleküls ausgewählt aus der Gruppe der
Carbonsäuren, aktivierten Ester, Acylazide, Acylhalogenide, Acylnitrile, Aldehyde, Anhydride, Arylamide, Alkylhalogenide, Aniline, Alkylsulfonate, Arylhalogenide, Thiole, Azide, Aziridine, Borate, Carbodiimide, Diazoalkane, Epoxide, Glycerole, Haloacetamine, Halotriazine, Hydrazine, Hydroxylamine, Imidoester, Isocyanate, Isothiocyanate, Maleimide, Phosphoramidite, Silylhalogene, Sulfonsäureester und Sulfonsäurechloride.

Die weiteren Reste sind unabhängig voneinander ausgewählt aus der Gruppe H, Alkyl (C₁-C₁₀), Alkoxy (C₁-C₁₀), Trifluormethyl, Halogen, Sulfonsäure, Sulfonat, Phosphorsäure und Phosphonat.

Durch die Verwendung von Cyaninfarbstoffen mit sperrigen Substituenten in 3-Position der Indolkopfgruppe, insbesondere cyclische Substituenten, sog. Spirane, z.B., Spiro-1'-Cyclohexan, Spiro-4'-1'-Piperidin, 4'-1'-Tetrahydrapyran, Spiro-4'-1'-tetrahydrothiopyran, Spiro-4'-1'-Oxotetrahydrothiopyran, Spiro-4'-1',1' Dioxotetrahydrothiopyran, kommt es zu einer weitgehenden Abschirmung der zentralen Polymethinkette.

Überraschenderweise konnte gemäß der Erfindung gezeigt werden, daß derartig substituierte Fluorochrome eine signifikant reduzierte Tendenz zur Aggregation besitzen. Darüber hinaus konnte eine höhere Photostabilität im Vergleich zu kommerziellen Cyaninfarbstoffen beobachtet werden. Besonders interessant ist hier die Verwendung von funktionalisierten cyclischen Substituenten, z.B. substituierte Spiro-Cyclohexane, Spiro-Piperidine oder Spiro-Tetrahydrothiopyrane. So wird eine weitere elegante Möglichkeit geschaffen, durch geeignete Reste sowohl die Wasserlöslichkeit durch Einführung polarer oder unpolarer Substituenten gezielt zu steuern, als auch das Bindungsverhalten des Farbstoffs durch Einführung reaktiver Gruppen Rₓ oder ionischer Gruppen positiv zu beeinflussen. Durch die Einführung weiterer sperriger Substituenten am Spiran wird die abschirmende und damit stabilisierende Wirkung der Spiranreste noch verstärkt.

Die erfindungsgemäßen Fluorochrome können durch Licht mit einer Wellenlänge von 600-1000 nm angeregt werden. Typischerweise besitzen die Fluorochrome hohe molare Extinktionskoeffizienten und sehr hohe Quantenausbeuten. Der Stokes Shift beträgt in der Regel mindestens 15 nm. Eine breite Anwendung dieser Farbstoffe, vor allem in der biochemischen Analytik, wird durch die Entwicklung kostengünstiger langwellig e-mittierender Laserdioden im Wellenlängenbereich von 670 bis 830 nm möglich.

In der allgemeinen Formel I kommt den Resten R₁, R₄, R₆ bis R₁₀, R₁₃ und R₁₇ bis R₂₁ eine besondere Bedeutung zu. Diese Positionen sind besonders geeignet um entweder Substituenten zur Erhöhung der Wasserlöslichkeit oder unpolare Substituenten zur Erhöhung der Lipophilie und/oder mindestens eine reaktive Gruppe Rₓ zum Koppeln an Biomoleküle einzuführen.

So weisen die Fluorochrome der allgemeinen Formel I bevorzugt für die Reste R₁, R₆ bis R₁₀, R₁₈ bis R₂₁, sowie besonders bevorzugt für die Reste R₆ bis R₉ und R₁₈ bis R₂₁, mindestens eine reaktive Gruppe Rₓ zum Koppeln an ein Zielmolekül auf.

Die reaktive Gruppe Rₓ kann direkt oder über eine aus mehreren Atomen bestehende Brücke kovalent mit dem Farbstoff verbunden sein und weist eine zum Koppeln geeignete Chemie auf. Bevorzugt wird die reaktive Gruppe Rₓ ausgewählt aus der Gruppe bestehend aus Succinimidylestern, Maleiimiden und Phosphoramiditen.

Nachfolgend sind beispielhafte Gruppen Rₓ mit ihren Strukturformeln dargestellt:
Nachfolgend sind beispielhafte Gruppen Rₓ mit ihren Strukturformeln dargestellt.

| | |
|---|---|
| aktivierte Carbonsäuren | |
| 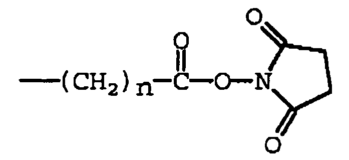 | |
| 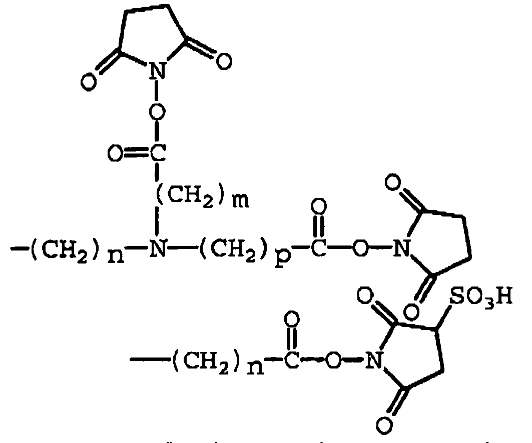 | |
| mit n, m und p unabhängig voneinander 1 bis 8 | |
| Carbodiimide | |
| | 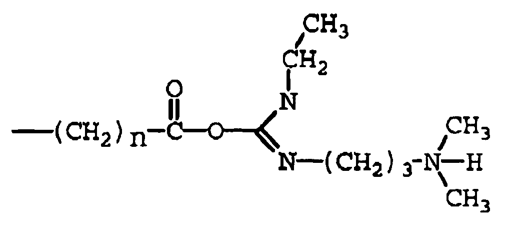 |
| mit n = 1 bis 8 | |
| Anhydride | |
| | 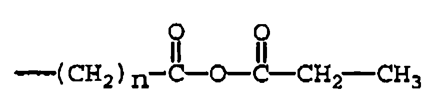 |
| mit n = 1 bis 8 | |
| Carbonsäureazide | |
| | 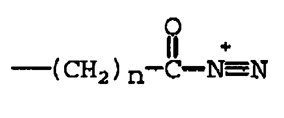 |
| mit n = 1 bis 8 | |
| Kopplung von Nukleophilen über Epoxide | |
| mit n = 1 bis 8 | 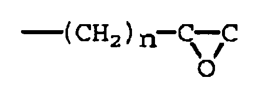 |
| Isothiocyanate | 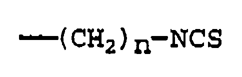 |
| | 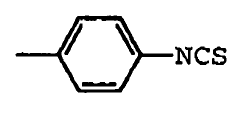 |
| mit n = 1 bis 8 Isocyanate | 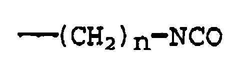 |
| mit n = 1 bis 8 | 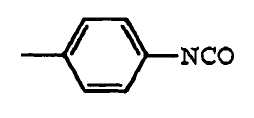 |
| Aziridine | 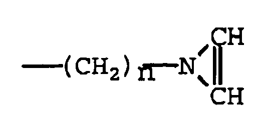 |
| mit n = 1 bis 8 | 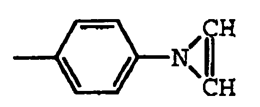 |
| Maleimide | 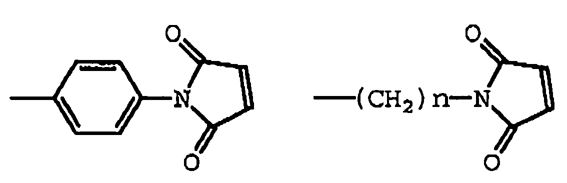 |
| mit n = 1 bis 8 | |
| Pyridyl-disulfid aktivierte Gruppen | |
| 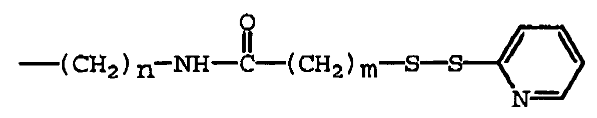 | |
| 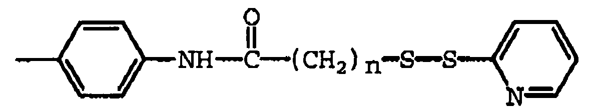 | |
| mit n und m unabhängig voneinander gleich 1 bis 8 | |
| Halodi- und triazine | |
| | 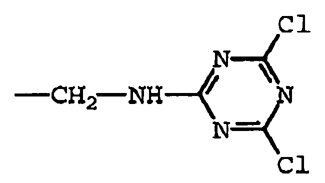 |
| | 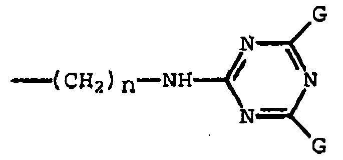 |
| mit G = Chlor oder Brom und n = 1 bis 8 Vinylsulfone | |
| mit n = 1 bis 8 | 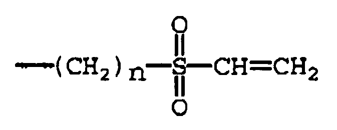 |
| Acylimidazole | |
| mit n = 1 bis 8 | 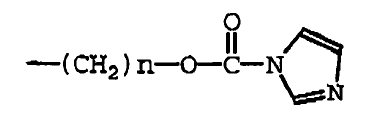 |
| Phosphoramidite | |
| mit n = 1 bis 8 | 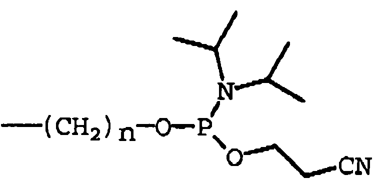 |

Tab. 1 zeigt eine Übersicht über mögliche Herstellungen von Farbstoffkonjugaten mit kovalenten Bindungen.

Zur Erhöhung der Wasserlöslichkeit können beispielhaft Carbonsäuren, Kohlenhydrate, Sulfonsäuren, Sulfonate, Phosphonate, Phosphate, Amine, Halogene, Polyole oder Polyäther ausgewählt und an jeder beliebigen Stelle des Fluoreezenzfarbstoffs angebracht werden, bevorzugt sind dabei mindestens einer der Reste R₁, R₄, R₆ bis R₁₀, R₁₃ und R₁₇ bis R₂₁, besonders bevorzugt mindestens einer der Reste R₁, R₆ bis R₁₀ und R₁₈ bis R₂₁, ganz besonders bevorzugt mindestens einer der Reste R₆ bis R₉ und R₁₈ bis R₂₁.

In einer weiteren vorteilhaften Weiterbildung des Verfahrens sind jeweils zwei am aromatischen Ring ortho-ständige Reste unter Ausbildung von mindestens einem aromatischen, carbo- oder heterocyclischen Ring verbrückt.

Der Farbstoff kann mittels einer oder mehrerer Gruppen Rₓ mit einem biologischen oder nicht biologischen Zielmolekül unter Ausbildung einer oder mehrerer kovalenten Bindung zu einem Komplex(Konjugat), wobei das Zielmolekül vorzugsweise aus der Gruppe Antikörper, Proteine, Peptide, Enzymsubstrate, Hormone, Lymphokine, Lipide, Phospholipide, Metabolite, Rezeptoren, Antigene, Haptene, Lecitine, Toxine, Kohlenhydrate, Oligosaccharide, Polysaccharide, Nukleinsäuren, Desoxyribo-nukleinsäuren, derivatisierte Nukleinsäuren, derivatisierte Desoxyribonukleinsäuren, DNA-Fragmente, RNA-Fragmente, Arzneimittel, Viruspartikel, Viruskomponenten, Hefen, Hefekomponenten, Bakterien, Bakterienkomponenten, Blutzellen, Blutzellenkomponenten, biologische Zellen, nichtzelluläre Blutkomponenten, Gifte, Polymere, Polymerteilchen, Glasteilchen, Glasoberflächen, Kunststoffoberflächen, Kunststoffteilchen, Polymermembran, Metalle, Leiter oder Halbleiter ausgewählt ist.

In einer vorteilhaften Weiterbildung wird das Fluorochrom an der Oberfläche von Nano- oder Micropartikeln, meist auf der Basis von Polymermaterialien, angebunden oder in diese Partikel eingeschlossen. Diese Partikel können dann anschließend in analytischen Verfahren vorteilhaft eingesetzt werden.

Die erfindungsgemäßen Fluorochrome zeichnen sich insbesondere dadurch aus, daß sie durch die Einführung der sperrigen Spiransubstituenten eine stark verringerte Aggregationsneigung aufweisen und somit einen hohen Kopplungsgrad am Zielmolekül ermöglichen, ohne Fluoreszenzlöschung durch Quencheffekte zu beobachten. Damit weisen sie eine hohe Quantenausbeute auf und sind so ausgezeichnet als Fluoreszenzfarbstoff, insbesondere als NIR-Fluoreszenzfarbstoff, für die Kopplung oder Bindung von Biomolekülen zur Verwendung in Bioassays geeignet. Die analytischen Verfahren umfassen alle Methoden, bei denen Biomoleküle mit Hilfe fluoreszenzoptischer Methoden detektiert werden. Eine bevorzugte Ausführungsform sind Fluoreszenzimmunotests, wobei verschiedenste bekannte biochemische Assays von allgemeinen Rezeptor-Ligand Systemen, wie z.B. Antikörper-Antigen, Lectin-Kohlenhydrat, DNA oder RNA-komplementäre Nukleinsäuren, DNA oder RNA-Protein, Hormonrezeptor, Enzym-Enzymcofaktoren, Protein G oder Protein A-Immunglvbulin oder Avidin-Biotin zugrunde gelegt werden.

Verfügt das erfindungsgemäße Fluorochrom über geeignete nukleophile Gruppen, vorzugsweise Amino-, Thio-, oder Hydroxygruppen ist eine Kopplung des Farbstoffs, z.B. nach Aktivierung als Phosphoramidit, an Nukleotide möglich. Dieses Verfahren ist besonders wichtig für die Herstellung farbstoffmarkierter (Desoxy-) Nukleotide, die u.a. in Sequenzierautomaten eingesetzt werden.

Im Folgenden werden einige bevorzugte erfindungsgemäße Fluorochrome anhand ihrer Strukturformel aufgeführt. 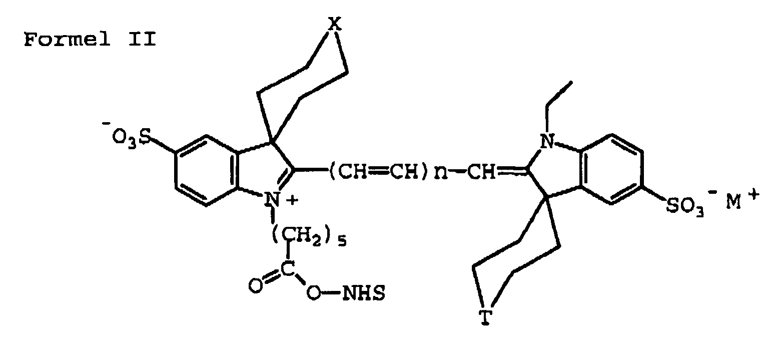 mit M⁺ = Metallkation sowie X, T, W⁻ und n entsprechend Anspruch 1 definiert. 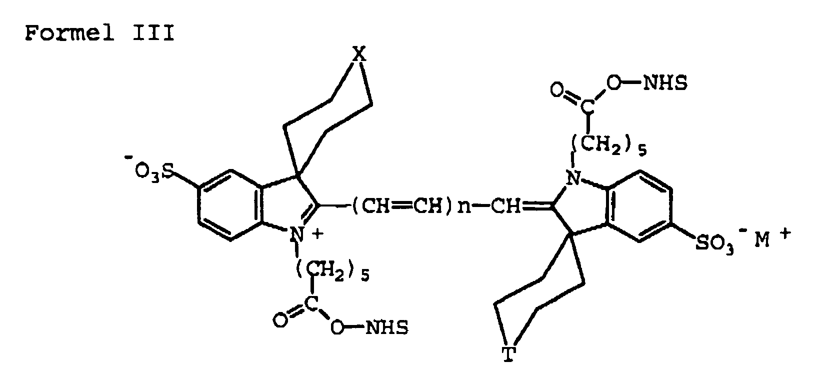 mit M⁺ = Metallkation, NHS = N-Hydroxy-succinimid sowie X, T, W⁻ und n entsprechend Anspruch 1 definiert. 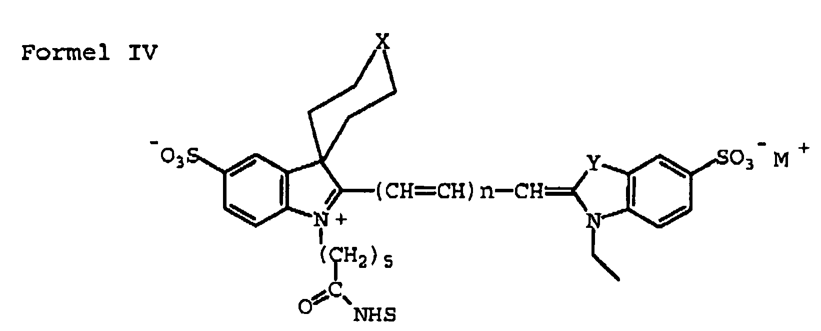 mit M⁺ = Metallkation, X, Y, T, W⁻, m und n entsprechend Anspruch 1 definiert. 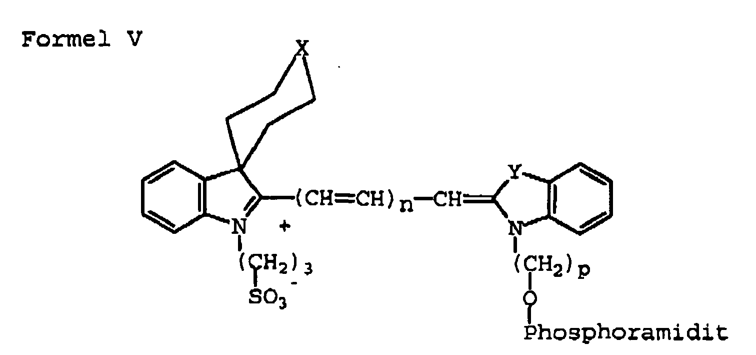 mit X, Y, T, W⁻, m und n entsprechend Anspruch 1 definiert, p = 1 - 8. 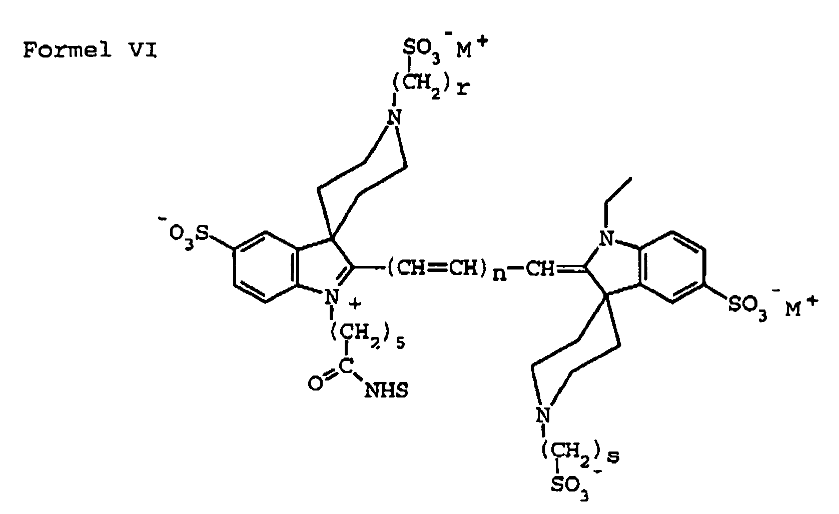 mit M⁺ = Metallkation und n entsprechend Anspruch 1 definiert, NHS = N-Hydroxy-succinimid, r und s unabhängig voneinander = 1 bis 8. 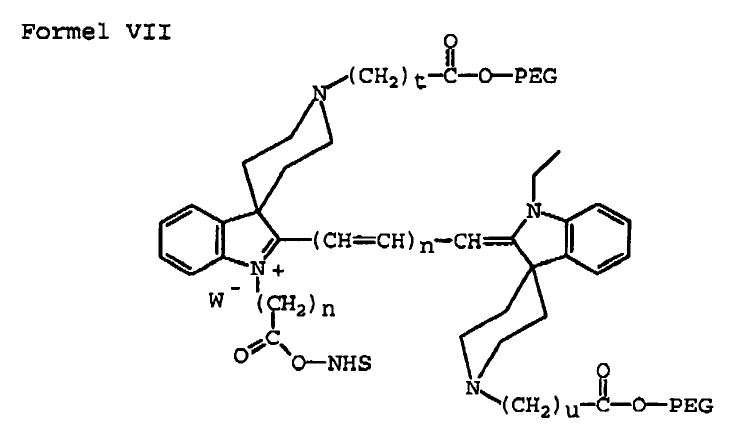 mit W⁻ und n entsprechend Anspruch 1 definiert, NHS = N-Hydroxy-succinimid, PEG = Polyethylenglykol sowie t und u unabhängig voneinander = 1 bis 8. 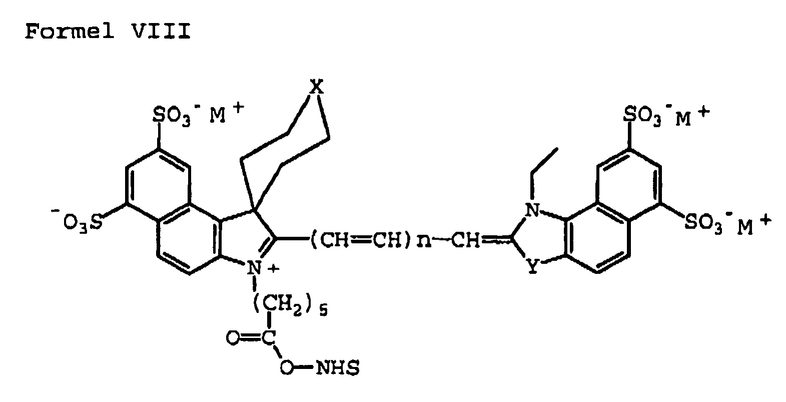 mit M⁺ = Metallkation, X, Y, T, W⁻ und n entsprechend Anspruch 1 definiert. 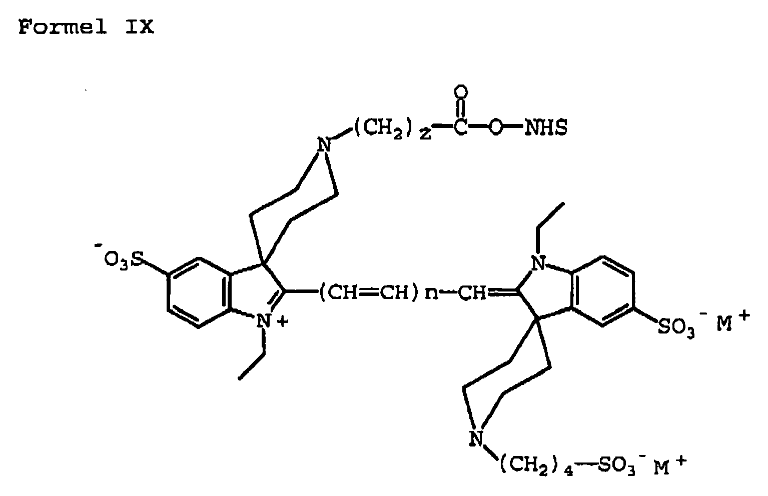 mit M⁺ = Metallkation, X, Y, T, W⁻ und n entsprechend Anspruch 1 definiert, z = 0 bis 6.

Der erfindungsgemäße Gegenstand soll nun anhand der folgenden Figuren und Beispiele näher erläutert werden, ohne diesen hierauf zu beschränken.
- Fig. 1: Synthese gemäß den Beispielen 1 bis 3 (Teil 1)
- Fig. 2: Synthese gemäß Beispielen 1 bis 3 (Teil 2)
- Fig. 3: Synthese gemäß Beispielen 4 bis 7 (Teil 1)
- Fig. 4: Synthese gemäß Beispielen 4 bis 7 (Teil 2)
- Fig. 5: Synthese gemäß Beispiel 8
- Fig. 6: Synthese gemäß Beispiel 9 (Teil 1)
- Fig. 7: Synthese gemäß Beispiel 9 (Teil 2)
- Fig. 8: Absorptionsspektren der erfindungsgemäßen Fluorochrome gemäß den Beispiel 1 bis 3 (Farbstoffe S1, S2 und S3).
- Fig. 9: Absorptions- und Emissionsspektrum des Fluorochroms gemäß Beispiel 2.
- Fig. 10: Absorptionsspektren des Proteinkonjugats gemäß Beispiel 10.
- Fig. 11: Photostabilität des Fluorophors S3 gemäß Beispiel 3
- Fig. 12: Fluoreszenz des Proteinkonjugate gemäß Beispiel 10 in Abhängigkeit von der Inkubationszeit.
- Fig. 13: Fluoreszenz in Gegenwart hoher Proteinkonzentrationen

### Herstellung von Farbstoffen der symmetrischen 3-Spiro-1'-Cyclohexanindolreihe

### Beispiel 1:

### Synthese Farbstoff S1

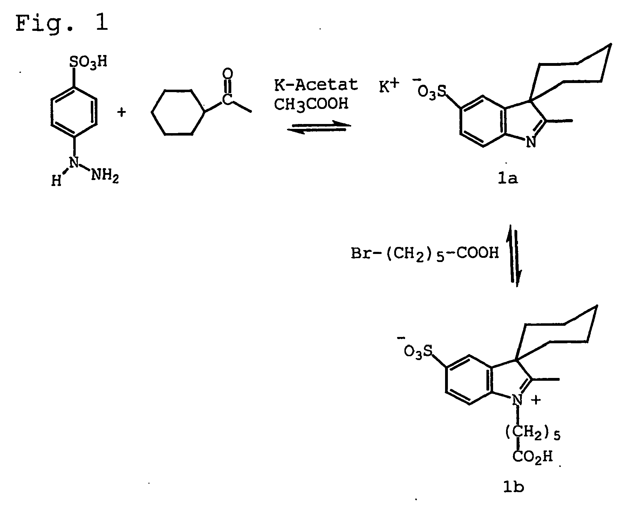

### Herstellung des 2-Methyl-3-Spiro-1'-Cyclohexan-5-sulfo-3H-indol; Kaliumsalz (Fig. 1, 1a)

30 mmol (5.646g) Phenylhydrazin-sulfonsäure werden mit 60mmol (7.572g) Cyclohexylmethylketon und 60mmol (5.889g) Kaliumacetat in 30ml Eisessig 6h unter Rückfluss erhitzt. Danach werden die flüchtigen Bestandteile im Vakuum abdestilliert. Der braune Rückstand wird in eine Extraktionshülse überführt und im Soxlethapparat mit 200ml 2-Propanol extrahiert. Das Produkt beginnt schon in der Wärme im Kolben auszukristallisieren, nach Erkalten wird abgesaugt und im Vakuum getrocknet.
Ausbeute: 6.044g (66% d.Th.)

### Herstellung des 1-(5-Carboxypentyl)-2-Methyl-3-Spiro-1'-Cyclohexan-5-sulfo-3H-Indolium Bromid; Kaliumsalz (1b)

4.5 mmol (1.418g) 2-Methyl-3-Spiro-1'-Cyclohexan-5-sulfoindolenin werden mit 5.5mmol (1.073g) 6-Bromhexansäure gemischt und in der Schmelze unter Rühren bei 110°C 2.5h zur Reaktion gebracht. Nach Erkalten wird das Produktgemisch mit Aceton digeriert, abgesaugt, mehrfach mit Aceton gewaschen und anschließend im Vakuum getrocknet.
Ausbeute: 1.505g (66% d. Th.)

Das quartärnisierte Spiro-Indol (2c) dient als Schlüsselbaustein für alle weiteren Synthesen

### Farbstoff S1

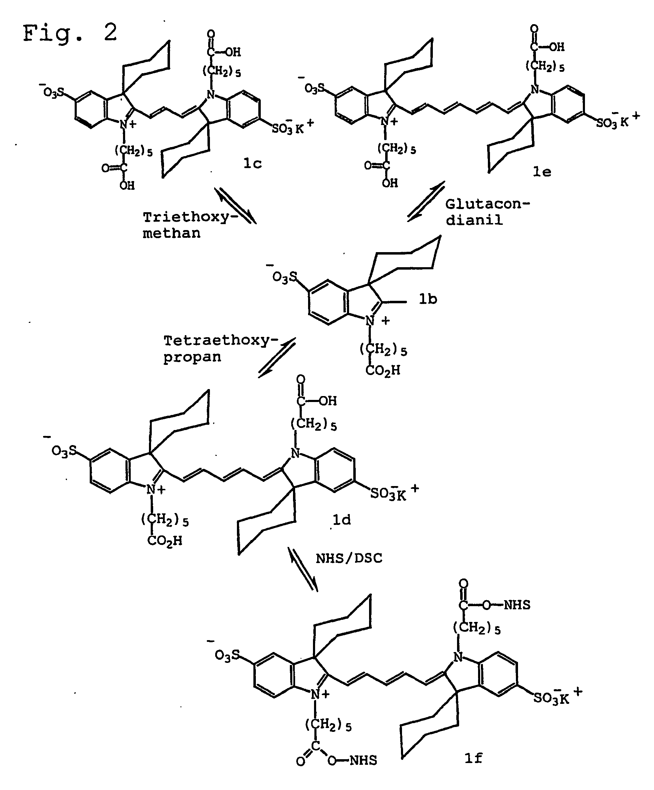

### 1-(5-Carboxypentyl)-2-[5-(1-(5-Carboxypentyl)-1,3-dihydro-3-spiro-1'-Cyclohexan-5-sulfo-2H-indol-2-yliden)-propenyl]-3-spiro-1'-Cyclohexan-5-sulfo-3H-indolium Hydroxid, Innersalz, Kaliumsalz: (Fig. 2, 1c)

0.4mmol (205mg) 1-(5-Carboxypentyl)-2-Methyl-3-Spiro-1'-Cyclohexan-5-sulfoindolenin (Kaliumsalz) werden in 2ml Pyridin mit 1mmol(148mg) Triethoxymethan versetzt und 30 min unter Rückfluss erhitzt. Nach Erkalten wird der Rohfarbstoff mit Ether gefällt und durch präparative HPLC mit einem Methanol-Wasser-Gradienten an Kieselgel RP-18 gereinigt.

Analog sind die längerkettigen Cyaninfarbstoffe zugänglich.

### Beispiel 2:

### Synthese Farbstoff S2

### 1-(5-Carboxypentyl)-2-[5-(1-(5-Carboxypentyl)-1,3-dihydro-3-spiro-1'-Cyclohexan-5-sulfo-2H-indol-2-yliden)-penta-1,3-dienyl]-3-spiro-1'-Cyclohexan-5-sulfo-3H-indolium Hydroxid, Innersalz, Kaliumsalz: (Fig. 2, 1d)

0.22mmol (111.6mg) 1-(5-Carboxypentyl)-2-Methyl-3-Spiro-1'-Cyclohexan-5-sulfoindolenin (Kaliumsalz) werden in 1ml trockenem Pyridin gelöst und unter Rückfluss erhitzt. Dann werden portionsweise über 2.5h verteilt 0.5ml 1.1.3.3-Tetraethoxypropan zugegeben. Der ausgeschiedene Farbstoff wird mehrmals mit Pyridin, dann mit Diethylether gewaschen und durch präparative HPLC mit einem Methanol-Wasser-Gradienten an Kieselgel RP-18 gereinigt.

### Beispiel 3:

### Synthese Farbstoff S3

### 1-(5-Carboxypentyl)-2-[5-(1-(5-Carboxypentyl)-1,3-dihydro-3-spiro-1'-Cyclohexan-5-sulfo-2H-indol-2-yliden)-hepta-1,3,5-trienyl]-3-spiro-1'-Cyclohexan-5-sulfo-3H-indolium Hydroxid, Innersalz, Kaliumsalz (Fig. 2, 1e)

0.32mmol (125mg) 1-(5-Carboxypentyl)-2-Methyl-3-Spiro-1'-Cyclohexan-5-sulfoindolenin (Kaliumsalz) werden mit 0.16mmol (45mg) Glutacondianilhydrochlorid und 0.65mmol (63mg) Kaliumacetat in einem Gemisch aus 2ml Essigsäureanhydrid und 0.5ml Eisessig 30min unter Rückfluss erhitzt. Der Rohfarbstoff wird mit Aceton gefällt, abgesaugt und durch präparative HPLC mit einem Methanol-Wasser-Gradienten an Kieselgel RP-18 gereinigt.

### Herstellung von Farbstoffen der unsymmetrischen 3-Spiro-1'-Cyclohexan-indolreihe

### Beispiel 4:

### Synthese Farbstoff S4

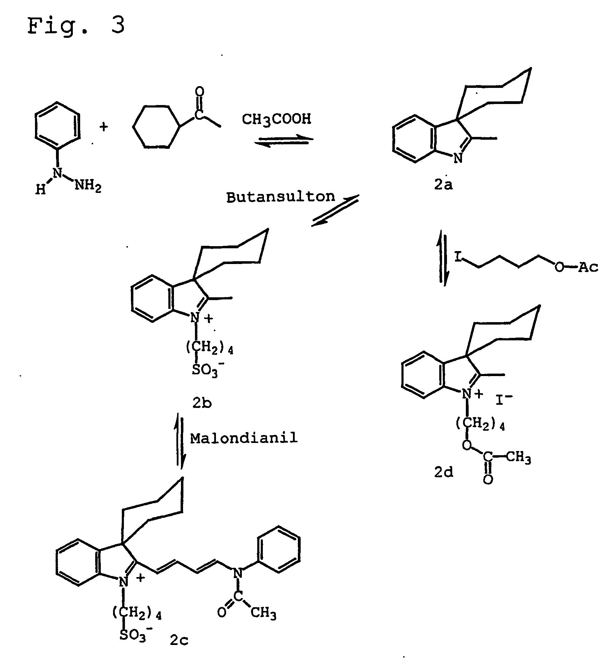

### 2-Methyl-3-spiro-1'-cyclohexan-3H-indol (Fig. 3, 2a)

1.25mol (135.2g)Phenylhydrazin und 1.5mol (189.3g) Cyclohexylmethylketon werden in 1.251 Eisessig 3h unter Rückfluss erhitzt. Der Eisessig wird im Vakuum verdampft, zum Rückstand 11 Wasser zugegeben und 4x mit Ether extrahiert. Nach Waschen der etherischen Phase mit Natriumhydrogencarbonat-Lösung und Trocknen mit Natriumsulfat, wird der Ether verdampft und der Rückstand im Vakuum destilliert.
Kp.(2mbar): 133-136°C
Ausbeute: 192.6g (77% d.Th.)

### 1-(4-Sulfobutyl)-2-methyl-3-spiro-1'cyclohexan-3H-indolium Hydroxid Innersalz (Fig. 3, 2b)

28.7mmol (5.72g) 2-Methyl-3-spiro-1'-cyclohexan-3H-indol und 32mmol (4.36g) Butansulton werden 4h auf 120°C erhitzt, Nach Erkalten wird mit 50ml Ether verrieben, 2x mit Ethylacetat, abermals mit Ether gewaschen und im Vakuum getrocknet.
Ausbeute: 7.93g (82% d.Th.)

### 2-(4-Acetanilino-1,3-butadienyl)-3-spiro-1'-cyclohexan-1-(4-sulfobutyl)-3H-indolium Hydroxid Innersalz (Fig. 3, 2c)

4mmol (1.34g) 1-(4-Sulfobutyl)-2-methyl-3-spiro1'cyclohexan-3H-indolium Hydroxid Innersalz werden mit 5mmol Malondianil (hergestellt aus 5mmol (1.62g) Malondianilhydroperchlorat und 5mmol (490mg) Kaliumacetat in abs. Ethanol, verdünnen mit Diethylether und einrotieren des Filtrats) in 5ml Essigsäureanhydrid und 5ml Essigsäure 3h auf 80° erwärmt. Danach wird das Lösungsmittel i.vak. verdampft und der Rückstand mit Ether digeriert, filtriert und mit Ether nachgewaschen bis der Ablauf farblos ist. Das Produkt wird im Vakuum getrocknet und ohne weitere Reinigung für die nächsten Stufen eingesetzt.
Ausbeute:2.00g (98%d.Th.)

### 1-(4-Acetoxybutyl)-2-Methyl-3-spiro-1'-cyclohexan-3H-indolium iodid (Fig. 3, 2d)

0.27mol (49.8g) 2-Methyl-3-spiro-1'-cyclohexan-3H-indol und 0.27mol (70.0g) 4-Iodbutylacetat werden zusammen 5h auf 110°C erhitzt. Nach Abkühlen wird in möglichst wenig Methylenchlorid gelöst und mit 500 ml Ether verrührt. Nach einiger Zeit unter Rühren kristallisiert das Produkt, wird scharf abgesaugt, mit Ether gewaschen und im Vakuum getrocknet.
Ausbeute: 104.9g (87% d.Th.)
Analog zur Herstellung des 1-(4-Acetoxybutyl)-2-Methyl-3-spiro-1'-cyclohexan-3H-indolium iodids (2d) sind die entsprechenden Derivate ausgehend von 2-Methylbenzoxazol, 2-Methylbenzothiazol, 4-Methylpyridin und 4-Methylchinolin zugänglich.

### 1-(4-Acetoxybutyl)-2-Methyl benzoxazolium iodid (2e)

10mmol (1.33g) 2-Methyl-Benzoxazol und 10mmol (2.42g) 4-Iodbutylacetat werden 6h auf 130°C erhitzt und nach Erkalen mit 10ml Ethylacetat verrieben. Die anfallenden Kristalle werden scharf abgesaugt, mit Ether nachgewaschen und im Vakuum getrocknet. Ausbeute:2.17g (58% d.Th.)

### 1-(4-Acetoxybutyl)-2-Methyl benzothiazolium iodid (2f)

10mmol (1.49g) 2-Methyl-Benzothiazol und 10mmol (2.42g) 4-Iodbutylacetat werden 4h auf 120°C erhitzt und nach Erkalten mit Ether verrieben scharf abgesaugt, mit Ether nachgewaschen und im Vakuum getrocknet.
Ausbeute:3.01g (77% d.Th.)

### 1-(4-Acetoxybutyl)-4-Methyl-pyridinium iodid (2g)

10 mmol (0.93g) γ-Picolin und 10 mmol (2.42g) 4-Iodbutylacetat werden 4h auf 120°C erhitzt und nach Erkalten mehrmals mit Ether verrührt, abdekantiert und anschliessend im Vakuum getrocknet. Auebeute:2.35g (70%d.Th.) viskose Masse

### 1-(4-Acetoxybutyl)-4-Methyl-chinolinium iodid (2h)

10mmol (1.43g) Lepidin und 10mmol (2.42g) 4-Iodbutylacetat werden 4h auf 120°C erhitzt und nach Erkalten mehrmals mit Ether verrührt, abdekantiert und anschliessend im Vakuum getrocknet. Ausbeute:3.13g (81%d.Th.) viskose Masse

### Farbstoff S4

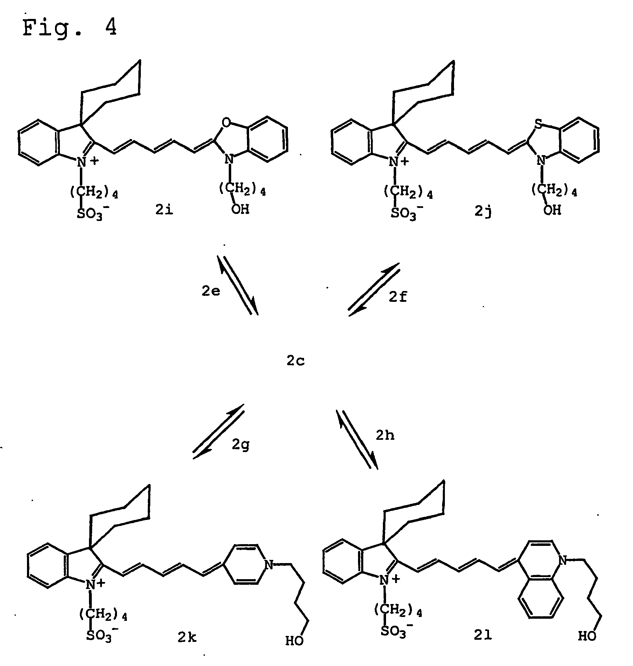

### 1-(4-Hydroxybutyl)-2-[5-(1,3-dihydro-1-(4-sulfobutyl)-3-spiro-1'-cyclohexan-2H-indol-2-yliden)-penta-1,3-dienyl]-benzoxazolium Hydroxid Innersalz (Fig. 4, 2i)

0.20mmol (101mg) 2-(4-Acetanilino-1,3-butadienyl)-3-spiro-1'cyclohexan-1-(4-sulfobutyl)-3H-indolium Hydroxid Innersalz, 0.21mmol (79mg) 1-(4-Acetoxybutyl)-benzoxazolium iodid und 0.21mmol (29.3µl) Triethylamin werden in 1ml abs. Ethanol 30min unter Rückfluss erhitzt. Nach Erkalten werden 5ml Ether zugegeben, und abdekantiert. Der Rückstand wird in einer Mischung aus 1ml konz. HCl und 9ml Methanol aufgenommen und über Nacht bei 4°C stehen gelassen. Das Lösemittel wird im Vakuum verdampft und der Rückstand an RP18-Kieselgel mit Acetonitril-Wasser (1:1) chromatographiert.

### Beispiel 5:

### Synthese Farbstoff S5

### 1-(4-Hydroxybutyl)-2-[5-(1,3-dihydro-1-(4-sulfobutyl)-3-spiro-1'-cyclohexan-2H-indol-2-yliden)-penta-1,3-dienyl]-benzothiazolium Hydroxid Innersalz (Fig. 4, 2j)

Analog obiger Vorschrift werden 0.21mmol (82mg) 1-(4-Acetoxybutyl)-benzothiazolium iodid mit den restlichen Reagenzien umgesetzt.

### Beispiel 6:

### Synthese Farbstoff S6

### 1-(4-Hydroxybutyl)-4-[5-(1,3-dihydro-1-(4-sulfobutyl)-3-spiro-1'-cyclohexan-2H-indol-2-yliden)-penta-1,3-dienyl]-pyridinium Hydroxid Innersalz (Fig. 4, 2k)

Analog obiger Vorschrift werden 0.21mmol (71mg) 1-(4-Acetoxybutyl)-pyridinium iodid mit den restlichen Reagenzien umgesetzt.

### Beispiel 7:

### Synthese Farbstoff S7

### 1-(4-Hydroxybutyl)-4-[5-(1.3-dihydro-1-(4-sulfobutyl)-3-spiro-1'-cyclohexan-2H-indol-2-yliden)-penta-1,3-dienyl]-Chinolinium Hydroxid Innersalz (Fig. 4, 2l)

Analog obiger Vorschrift werden 0.21mmol (81mg) 1-(4-Acetoxybutyl)-pyridinium iodid mit den restlichen Reagenzien umgesetzt.

Die nach 2i bis 21 hergestellten Hydroxysubstituierten Fluorophore lassen sich nach dem Stand der Technik mit 2-Cyanoethyl-N,N-Diisopropylchlorophosphoramidit in das entsprechende Phosphoramidit zum Koppeln an DNA umwandeln.

### Herstellung von Farbstoffen der unsymmetrischen 3-Spiro-1'-4-tetrahydropyran-indolreihe

### Beispiel 8:

### Synthese Farbstoff S8

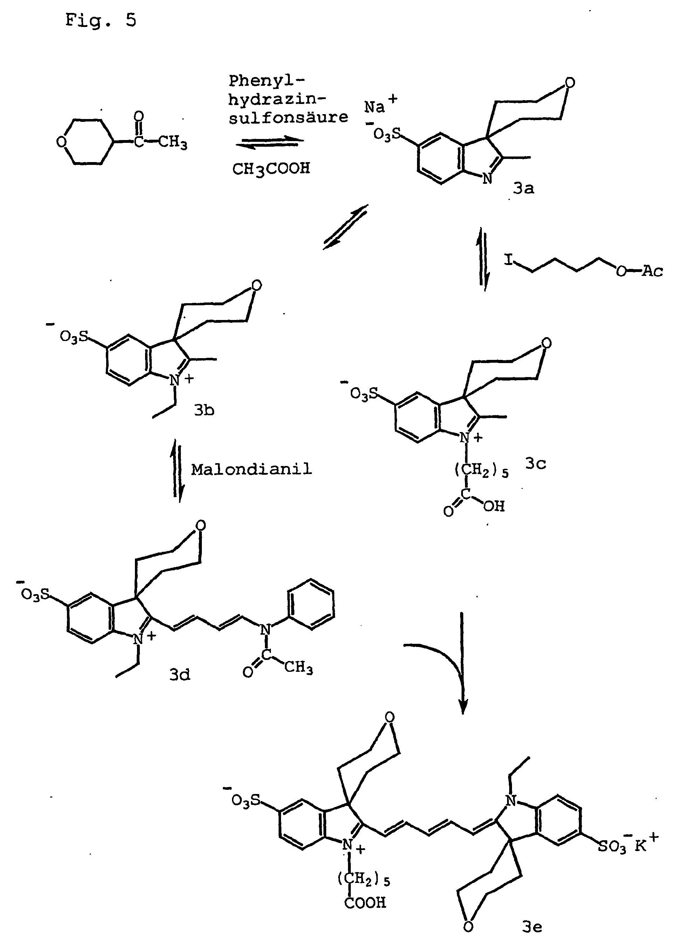

### 2-Methyl-5-sulfo-3-spiro-4'-tetrahydropyran-3H-indol (Fig. 5, 3a)

40mmol (7.53g) Phenylhydrazin-4-sulfonsäure und 44mmol 4-Acetyltetrahydropyran werden in 40ml Eisessig 48h unter Stickstoff zum Rückfluss erhitzt. Danach wird filtriert, die Essigsäure verdampft, in wenig Wasser aufgenommen und mit 4ml 10M NaOH versetzt. Nach Eindampfen zur Trockene wird der Rückstand mit 2-Propanol extrahiert der Extrakt einrotiert und i. Vak. getrocknet.
Ausbeute: 6.94g (57% d.Th.)

### 1-Ethyl-2-methyl-5-sulfo-3-spiro-4'-tetrahydropyran-3H-indolium Hydroxid Innersalz (Fig. 5, 3b)

5.2mmol (1,58g) 2-Methyl-5-sulfo-3-spiro-4'-tetrahydropyran-3H-indol und 5.72mmol (882mg) Diethylsulfat werden in 3ml 1.2-Dichlorbenzol 5h auf 110°C erhitzt. Nach dem Abkühlen wird mit Aceton verdünnt und filtriert. Der Filterkuchen wird intensiv mit Aceton nachgewaschen und im Vakuum getrocknet. Ausbeute:1.08g (67%d.Th.)

### 1-(5-Carboxypentyl)-2-methyl-5-sulfo-3-spiro-4'-tetrahydropyran-3H-indolium Innersalz (Fig. 5, 3c)

10mmol (3.03g) 2-Methyl-5-sulfo-3-spiro-4'-tetrahydropyran-3H-indol und 15mmol (22.93g) 6-Bromhexansäure werden in 10ml 1,2-Dichlorbenzol 24h auf 120°C erhitzt, Nach Erkalten wird mit Aceton verdünnt und abdekantiert. Danach wird mit heissem 2-Propanol digeriert, abgekühlt, filtriert, mit 2-Propanol und Aceton nachgewaschen und abschliessend i. Vak. getrocknet. Ausbeute: 3.07g (78%.d.Th.)

### 2-(4-Acetanilino-1,3-butadienyl)-1-ethyl-3-spiro-4'-tetrahydropyran-5-sulfo-3H-indolium Hydroxid Innersalz (Fig. 5, 3d)

3mmol (0.93g) 1-Ethyl-2-Methyl-3-spiro-4'-tetrahydropyran-5-sulfo-3H-indolium Toluolsulfonat werden mit 3.6mmol Malondianil (hergestellt aus 3.6mmol (1.165g Malondianilhydroperchlorat und 3.6mmol (353mg) Kaliumacetat in abs. Ethanol, verdünnen mit Diethylether und einrotieren des Filtrats) in 15ml Essigsäure/Essigsäureanhydrid (1:1 v/v) 3h auf 80°C erhitzt. Anschließend wird das Lösungsmittel i. Vak. Verdampft und der Rückstand mit Ether digeriert, abgesaugt, mit Ether gewaschen bis der Ablauf farblos ist und i. Vak. getrocknet.
Ausbeute:1.33g (92% d.Th.)

### Farbstoff S8

### 1-(5-Carboxypentyl)-2-[5-(1,3-dihydro-1-ethyl)-3-spiro-4'-tetrahydropyran-5-sulfo-2H-indol-2-yliden)-penta-1,3-dienyl]-3-spiro-4'-tetrahydropyran-5-sulfo-3H-indolium Hydroxid Innersalz Kaliumsalz, (Fig. 5, 3e)

2.5mmol (1.20g) 2-(4-Acetanilino-1,3-butadienyl)-3-spiro-4'-tetrahydropyran-1-ethyl-5-sulfo-3H-indolium Hydroxyd Innersalz werden in einer Mischung aus 5ml Pyridin und 5ml Essigsäureanhydrid mit 2.7mmol (1.07g) 1-(5-Carboxypentyl)-2-methyl-5-sulfo-3-spiro-4'-tetrahydropyran-3H-indolium Innersalz versetzt und 40 min zum Rückfluss erhitzt. Es wird im Vakuum auf ca. ein Drittel eingeengt und der Rohfarbstoff mit Ether gefällt und abdekantiert. Der Rückstand wird in der minimalen Menge 1M HCl gelöst und durch Zugabe von gesättigter KCl-Lösung ausgefällt. Das Präzipitat wird abgetrennt, in Wasser gelöst und an RP18-Kieselgel und Acetonitril-Wasser-Gradienten chromatographiert.

### Herstellung von Farbstoffen der unsymmetrischen 3-Spiro-1'-4-piperidyl-indolreihe

### Beispiel 9:

### Synthese Farbstoff S9

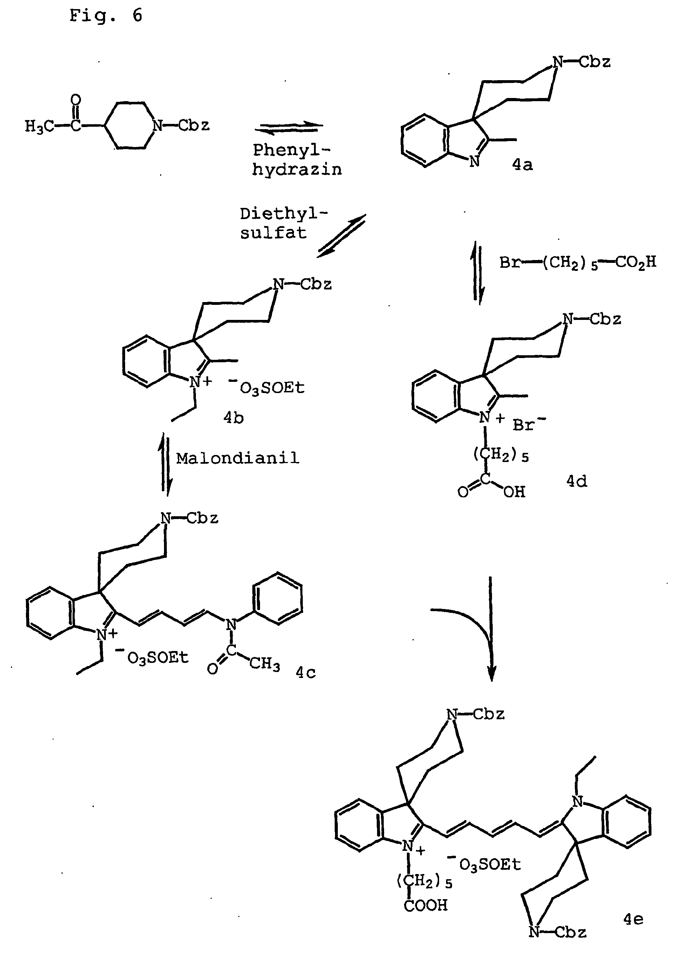

### Synthese von 2-Methyl-3-spiro-4'-(1'-carboxybenzyl)-piperidin-3H-indol (Fig. 6, 4a)

4.31 g (16.5 mmol) des Piperidylketons und 1.62 g (1.48 ml, 15 mmol) Phenylhydrazin werden unter Schutzgas-atmosphäre in 15 ml Eisessig gelöst und 3.5 h zum Sieden erhitzt. Die überschüssige Essigsäure wird am Rotationsverdampfer unter vermindertem Druck abdestilliert, wobei ein braunes Ö1 zurückbleibt. Das Ö1 wird in 80 ml Wasser aufgenommen und drei mal mit je 40 ml Diethylether extrahiert. Die etherischen Phasen werden vereinigt, noch einmal mit 40 ml Wasser gewaschen und über Magnesiumsulfat getrocknet. Das Lösungsmittel wird im Vakuum am Rotationsverdampfer abdestilliert. Das zurückbleibende, braune Öl wird säulenchromatographisch gereinigt (Dichlormethan /Ethylacetat = 2:1). Man erhält das Indol als rötliches Ö1.
Ausbeute = 3.23 g, 9.7 mmol, 64 % d. Th.

### Synthese von 1-Ethyl-2-methyl-3-spiro-4'-(1'-carboxybenzyl)-piperidin-3H-indolenium Ethylsulfat (Fig. 6, 4b)

Unter Schutzgasatmosphäre werden 2.06 g (6 mmol) des 2-Methyl-3-spiro-4'-(1'-carboxybenzyl)-piperidin-3H-indol (4a) in 3 ml Toluol gelöst. Mit einer Spritze werden 1.02 g (0.89 ml, 6.6 mmol) Diethylsulfat zugegeben. Die Reaktionsmischung wird 3.5 h am Rückfluß erhitzt, wobei sich ein dunkel-violettes Öl abscheidet, das nach Erkalten fest wird. Die überstehende Lösung wird abdekantiert und der Rückstand wird mehrfach mit kleinen Portionen Toluol gewaschen. Der Feststoff wird säulenchromatographisch mit Hilfe eines Lösungsmittelgradienten gereinigt (1. Ethylacetat, 2. Aceton/Eisessig = 10:2). Das quartärnierte Inden wird mit Lösungsmittel zweimal eluiert. Man erhält das Produkt als rötliches, hochviskoses Öl (0.93 g, 1.9 mmol, 32 % d. Th.).

### Synthese von 2-(4-Acetanilino-1,3-butadienyl)-1-ethyl-3-spiro-4'-(1'-carboxybenzyl)-piperidin-3H-indolenium Ethylsulfat (Fig. 6, 4c)

0.93 g (1.9 mmol) des quartärnierten Indols (4b) werden mit 2.4 mmol Malondianil (hergestellt aus 0.79 g (2.4 mmol) Malondianilhydroperchlorat und 0.23 g (2.4 mmol) Kaliumacetat in abs. Ethanol, verdünnen mit Diethylether und einrotieren des Filtrats) in 3 ml Essigsäureanhydrid und 3 ml Essigsäure 3 h auf 80 °C erwärmt. Das Lösungsmittel wird unter vermindertem Druck am Rotationsverdampfer abdestilliert. Der feste Rückstand wird mit Diethylether digeriert, abfiltriert und mit Diethylether nachgewaschen, bis die waschlösung farblos bleibt. Das Rohprodukt (1.19 g, 1.8 mmol, 97 % d. Th.) wird im Vakuum getrocknet und ohne weitere Reinigung in der nächsten Stufe eingesetzt.

### Synthese von 1-Carboxypentyl-2-methyl-3-spiro-4'-(1'-carboxybenzyl)-piperidin-3H-indolium Bromid (Fig. 6, 4d)

2.06 g (6 mmol) des 2-Methyl-3-spiro-4'-(1'-carboxybenzyl)-piperidin-3H-indol (4a) werden in 5 ml 6-Bromhexansäure für 3.5 h auf 120 °C Badtemperatur erhitzt. Die überstehende Lösung wird vom festen, rötlichen Rückstand abdekantiert. Die Reinigung des Rohproduktes erfolgt säulenchromatographisch (Wasser/Eisessig = 10:2). Das quartärnierte Indols (4c) wird als rötliches, hochviskoses Öl (0.95 g, 1.8 mmol, 30 % d. Th.) erhalten.

### Farbstoff S 9

### Synthese von 1-(5-Carboxypentyl)-2-[5-(1,3-dihydro-1-(1-ethyl)-3-spiro-4'-4'-(1'-carboxybenzyl)-piperidin-2H-indol-2-yliden)-penta-1,3-dienyl]-3-spiro-4'-4'-(1'-carboxybenzyl)-piperidin-3H-indolenium Ethylsulfat (Fig. 6, 4e)

1.19 g (1.8 mmol) des Hemicyanins (4c) und 0.83 g (1.8 mmol) des Indols (4d) werden in 3 ml Essigsäureanhydrid und 3 ml Pyridin 30 min unter Rückfluß erhitzt. Die Reaktionsmischung wird im Vakuum am Rotationsverdampfer zur Trockene eingedampft. Der feste Rückstand wird in einer Mischung aus 10 ml HCl konz. und 100 ml Methanol aufgenommen und über Nacht bei 4 °C stehen gelassen. Das Lösungsmittel wird unter vermindertem Druck abdestilliert und der Rückstand säulenchromatographisch gereinigt (Isopropanol/Wasser 10:2, v/v). Man erhält den Farbstoff S9 als dunkelblauen, kristallinen Feststoff (0.37 g, 0.38 mmol, 21 % d. Th.).

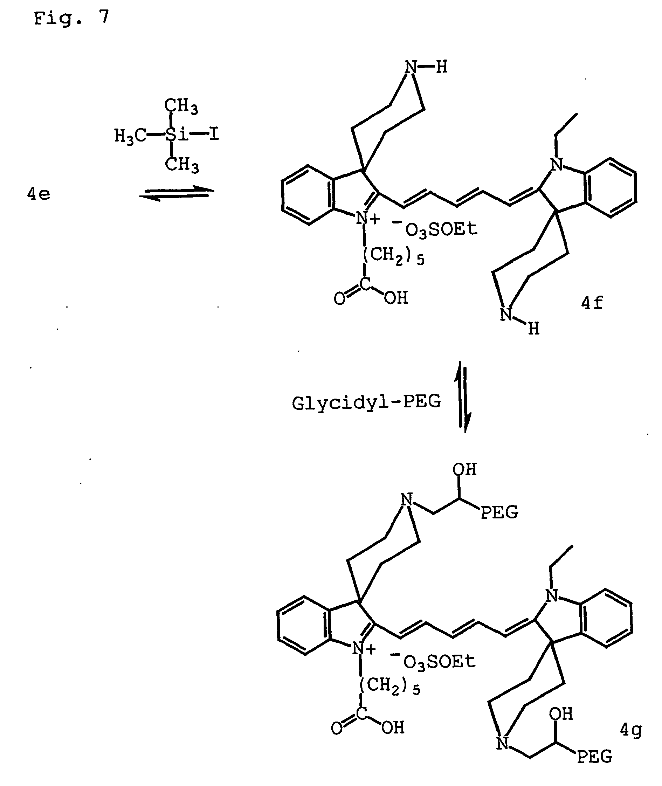

### Synthese von 1-(5-Carboxypentyl)-2-[5-(1,3-dihydro-1-(1-ethyl)-3-spiro-4'-4'-piperidin-2H-indol-2-yliden)-penta-1,3-dienyl]-3-spiro-4'-4'-piperidin-3H-indolenium Ethylsulfat (Fig. 7, 4f)

0.37 g (0.38 mmol) des Farbstoffs (4e) werden unter Schutzgasatmosphäre in 5 ml Chloroform gelöst. Mit einer Spritze werden 0.09 g (0.07 ml, 0.46 mmol) I-odtrimethylsilan zugegeben und die Mischung bei Raumtemperatur gerührt, bis dünnschicht-chromatographisch (Isopropanol/Wasser = 10:2, v/v) kein Edukt mehr nachweisbar ist. Dann werden 2 ml Methanol zugegeben und die Mischung wird 5 min bei Raumtemperatur gerührt. Die flüssigen Komponenten werden unter vermindertem Druck am Rotationsverdampfer abdestilliert. Der feste Rückstand wird säulenchromatographisch im Laufmittel Isopropanol/Wasser = 2:1 gereinigt. Ausbeute: 0.21 g, 0,3 mmol, 80 % d. Th.

Der entschützte Farbstoff S9 (4f) läßt sich durch Aufschmelzen mit Gylcidyl-PEG in einen PEGsubstituierten Farbstoff (4g) umwandeln.

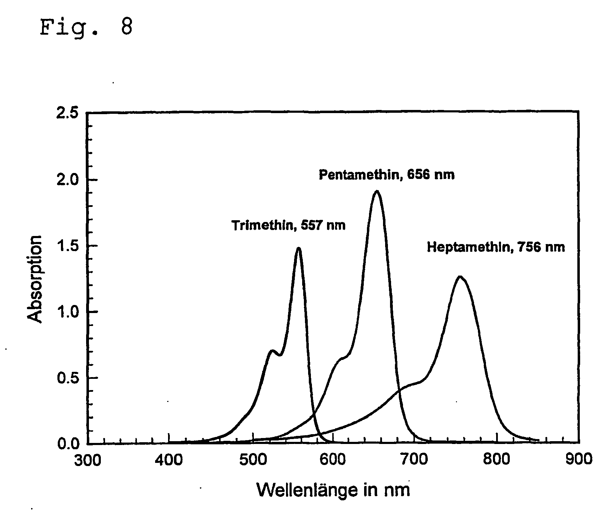

Die Absorptionsspektren der in Ausführungsbeispiel 1-3 hergestellten Farbstoffe (S1-S3) sind in Fig. 8 dargestellt.

Zur Kopplung an Zielmoleküle (M) müssen die Carbonylfunktionen in aktivierte Ester (Reaktive Gruppe Rₓ) überführt werden. Dies kann nach dem Stand der Technik mit Hilfe von N-Hydroxysuccinimid (NHS) in Gegenwart von 1-Ethyl-3-(3-dimethylamiriopropyl) carbodiimide (EDC) in wäßriger Lösung oder mittels Dieyclohexylcarbodiimid (DCC) in organischer Lösung geschehen (Fig 2, 1f).

### Beispiel 10

### Kopplung des NHS aktivierten Pentamethin-Spirocyanins (S2) an Protein

Zu 1 mg Rinderserumalbumin in 1 ml 10 mM Phosphatpuffer, pH 8,0, werden unter Schütteln 0,2 mg aktivierter Farbstoff gegeben. Nach einer Reaktionszeit von 60 min, wird die Kopplung durch Zugabe von 40 µl Glycinlösung (10% m/v, in PBS-Puffer, pH 7,4) abgestoppt und der überschüssige Farbstoff durch Dialyse oder Gelfiltration entfernt.

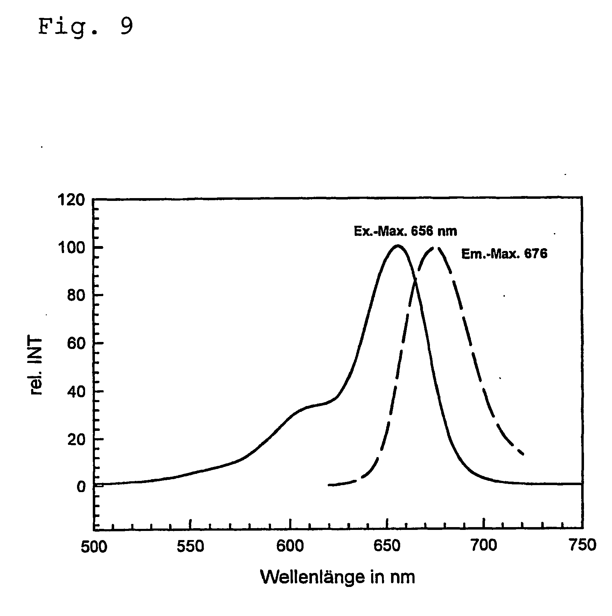

In Fig. 9 ist ein Emissionsspektrum des in Beispiel 2 und 10 beschriebenen Farbstoff-Konjugats gezeigt.

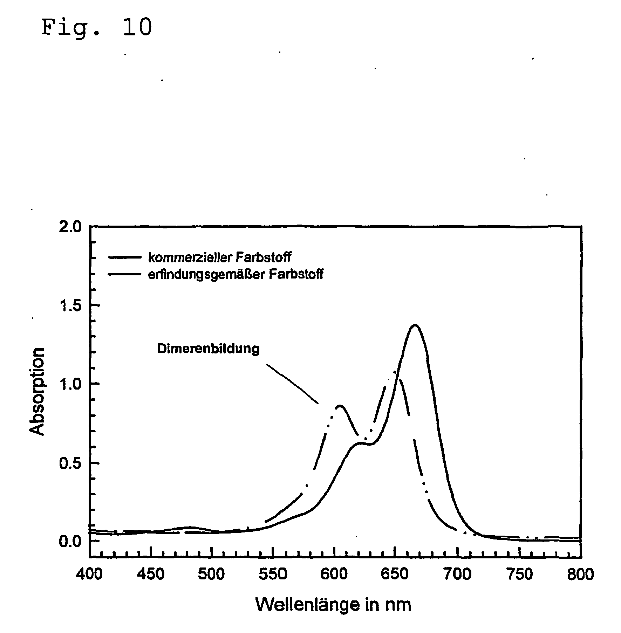

Im Vergleich dazu zeigt Fig. 10 entsprechende Spektren eines herkömmlichen, unter identischen Bedingungen hergestellten Konjugats. Deutlich ist hier eine bei Absorptionsspektrum, durch Dimerbildung hervorgerufene Bande bei ca. λ = 600 nm zu sehen, die zu einer verminderten Fluoreszenz führt.

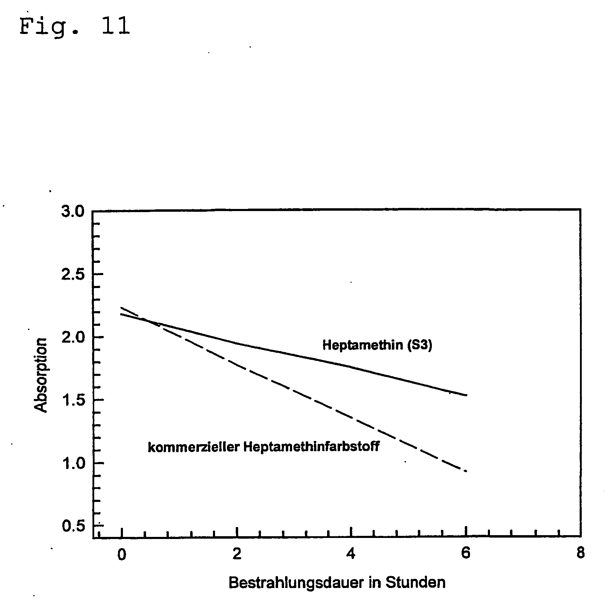

Durch die Wirkung der Spiransubstituenten kommt es unter anderem zu einer verminderten Beweglichkeit des Fluorophors, was sich in einer gesteigerten Photostabilität äußert. Fig. 11 zeigt die Photostabilität des Fluorophors S3 gemäß Ausführungsbeispiel 3, bestimmt in 10 mM Phosphatpuffer, pH 7,4 im Vergleich zu einem kommerziellen Heptamethinfarbstoff nach dem Stand der Technik. Bestrahlung wurde mit einer Krypton-Lampe mit 20 mW/cm2 bei λ > 400 nm.

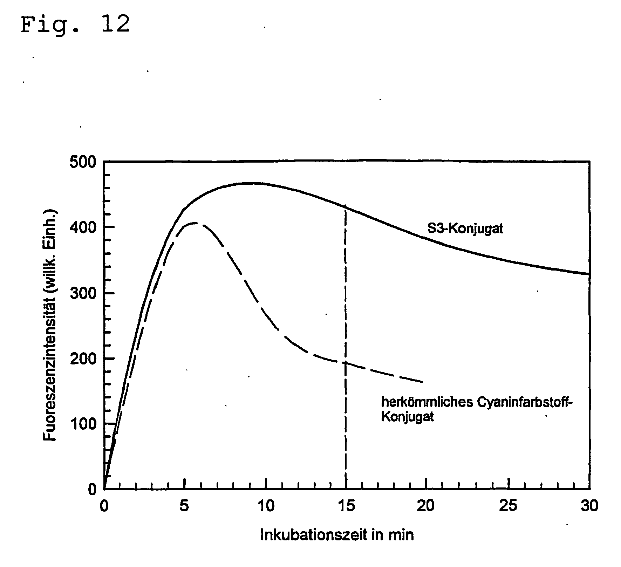

Bestimmt man die Fluoreszenz des S3-Konjugats, gemäß Ausführungsbeispiel 10 in Abhängigkeit von der Inkubationszeit mit dem Fluorophor, im Vergleich zu einem Konjugat, hergestellt mit einem herkömmlichen Cyaninfarbstoff, erhält man das in Fig. 12 dargestellte Bild. Gemessen wurde die resultierende Fluoreszenz der Konjugate, hergestellt unter identischen Bedingungen nach Abtrennung des überschüssigen Farbstoffs bei verschiedenen Inkubationszeiten. Je länger die Inkubationszeiten, desto höher ist der Kopplungsgrad Fluorophor:Protein. Beim herkömmlichen Konjugat kommt es zur Ausbildung eines schmalen Maximums, d.h. bei höheren Labelgraden sinkt die Fluoreszenz um mehr als 50 % durch Quencheffekte. Im Gegensatz dazu zeigt das Konjugat mit dem erfindungsgemäßen Farbstoff (Beispiel 2 u. 10) eine wesentlich reduzierte Aggregationsneigung und damit eine höhere Fluoreszenz. Nach 15 minütiger Inkubation ist die Fluoreszenz des Konjugats mit dem erfindungsgemäßen Fluorophor (Beispiel 10) mehr als doppelt so hoch wie die, des Konjugats mit dem Fluorophor nach dem Stand der Technik.

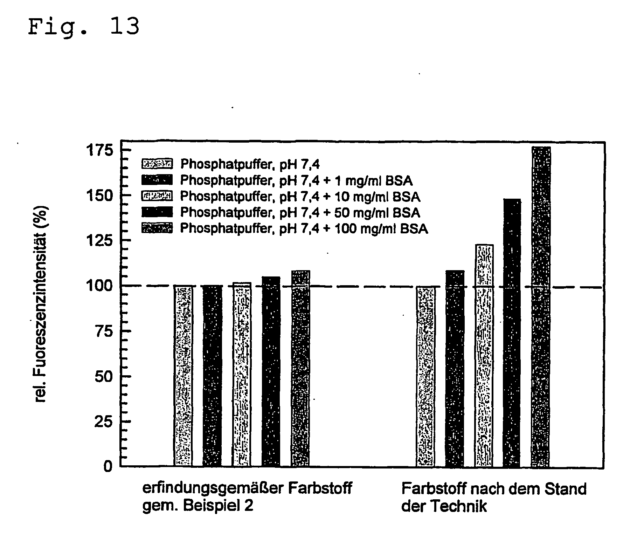

Ebenfalls durch die Spirosubstituenten hervorgerufen ist eine Abschirmung der zentralen Polymethinkette gegenüber Einflüssen durch die Umgebung des Fluorophors. Bekanntermaßen kommt es bei vielen Fluorophoren durch die Erhöhung der Proteinkonzentration einerseits zu einer langwelligen Verschiebung des Absorptionsmaximums und gleichzeitig zu einer Erhöhung der Fluoreszenz. Dies ist verursacht durch die geänderte Umgebung, hervorgerufen durch durch das Protein und die reduzierte Beweglichkeit des Fluorochroms. Beim erfindungsgemäßen Farbstoff wird dieser Effekt minimiert. Wie in Fig. 13 (normierte Darstellung) gezeigt, kommt es beim erfindungsgemäßen Fluorophor nur zu einer geringfügigen Erhöhung der Fluoreszenz in Gegenwart hoher Konzentrationen an Rinderserumalbumin (BSA). Im Gegensatz dazu ist die Fluoreszenz des kommerziellen Fluorophors stark von der Umgebung abhängig. Letzterer Effekt ist von großem Nachteil bei der Verwendung von Fluoreszenzmarkern bei der Untersuchung von Substanzen in Blutseren.

Tab. 2 zeigt die spektralen Charakteristika einiger erfindungsgemäßer Farbstoffe.

**Tab. 1:**

| Elektrophile Gruppe | Nukleophile Gruppe | Entstehender Bindungstyp |
|---|---|---|
| aktivierte Ester ¹⁾ | Amine/Aniline | Carboxamide |
| Acylazide | Amine/Aniline | Carboxamide |
| Acylhalogene | Amine/Aniline | Carboxamide |
| Acylhalogene | Alkohole/Phenole | Ester |
| Acylnitrile | Alkohole/Phenole | Ester |
| Acylnitrile | Amine/Aniline | Carboxamide |
| Aldehyde | Amine/Aniline | Imine |
| Aldehyde/Ketone | Hydrazine | Hydrazone |
| Aldehyde/Ketone | Hydroxylamine | Oxime |
| Alkylhalogene | Amine/Aniline | Alkylamine |
| Alkylhalogene | Carbonsäuren | Ester |
| Alkylhalogene | Thiole | Thioether |
| Alkylhalogene | Alkohole/Phenole | Ether |
| Alkylsulfonate | Thiole | Thioether |
| Alkylsulfonate | Carbonsäuren | Ester |
| Alkylsulfonate | Alkohole/Phenole | Ester |
| Anhydride | Alkohole/Phenole | Ester |
| Anhydride | Amine/Aniline | Carboxamide |
| Arylhalogene | Thiole | Thiophenole |
| Arylhalogene | Amine | Arylamine |
| Aziridine | Thiole | Thioester |
| Boronate | Glykole | Boronatester |
| Carbonsäuren | Amine/Aniline | Carboxamide |
| Carbonsäuren | Alkohole | Ester |
| Carbonsäuren | Hydrazine | Hydrazide |
| Carbodiimide | Carbonsäuren | N-Acylharnstoffe |
| Diazoalkane | Carbonsäuren | Ester |
| Epoxide | Thiole | Thioether |
| Haloacetamide | Thiole | Thioether |
| Halotriazine | Amine/Aniline | Aminotriazine |
| Halotriazine | Alkohole/Phenole | Triazinether |
| Imidoester | Amine/Aniline | Amidine |
| Isocyanate | Amine/Aniline | Harnstoffe |
| Isocyanate | Alkohole/Phenole | Urethane |
| Isothiocyanate | Amine/Aniline | Thioharnstoffe |
| Maleimide | Thiole | Thioether |
| Phosphoramidite | Alkohole | Phosphitester |
| Silylhalogene | Alkohole | Silylether |
| Sulfonsäureester | Amine/Aniline | Alkylamine |
| Sulfonsäureester | Carbonsäuren | Ester |
| Sulfonsäureester | Thiole | Thioether |
| Sulfonsäureester | Alkohole | Ether |
| Sulfonsäurechloride | Amine/Aniline | Sulfonamide |
| Sulfonsäurechloride | Alkohole/Phenole | Sulfonsäureester |

| | | |
|---|---|---|
| ¹⁾ aktivierte Ester sind Ester der Formel -CO-W, wobei W eine geeignete Abgangsgruppe, z.B., Nitro, Fluoro, Chloro, Cyano, Trifluormethyl, Tosyl etc. ist | | |

**Tab. 2**

| Farbstoff | Absorptions maximum | Emissions-maximum |
|---|---|---|
| | (nm) | (nm) |
| S1* | 557 | 572 |
| S2* | 656 | 672 |
| S3* | 756 | 772 |
| S4** | 612 | 638 |
| S5** | 650 | 676 |
| S6** | 603 | 626 |
| S7** | 702 | 728 |
| S8** | 662 | 684 |
| S9** | 660 | 682 |

| | | |
|---|---|---|
| * aufgenommen in Phopshatpuffer, pH 7.4 ** aufgenommen in Ethanol | | |

## Patentansprüche

1. Fluorochrom der allgemeinen Formel I 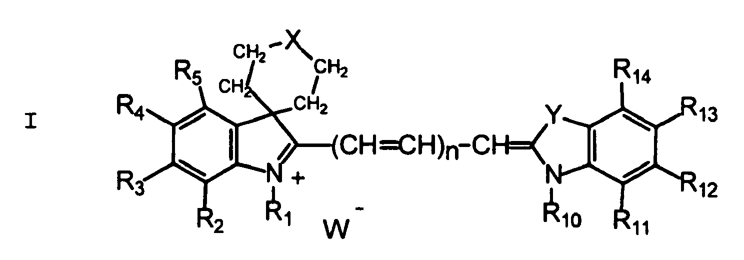 mit X = 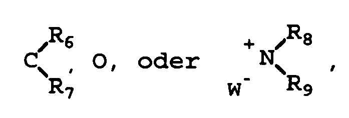 und Y ist, O, NR₁₇, 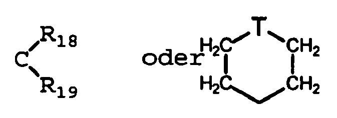 mit T gleich 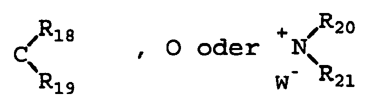 mit n = 0, 1, 2 oder 3 und
W⁻ = Gegenion, vorzugsweise Halogenid, Perchlorat oder Tosylat,
wobei mindestens einer der Reste R₁ bis R₂₁ ausgewählt ist aus
einer reaktiven Gruppe zur kovalenten Ankopplung eines Zielmoleküls ausgewählt aus der Gruppe der Carbonsäuren, aktivierten Ester, Acylazide, Acylhalogenide, Acylnitrile, Aldehyde, Anhydride, Arylamide, Alkylhalogenide, Aniline, Alkylsulfonate, Arylhalogenide, Thiole, Azide, Aziridine, Borate, Carbodiimide, Diazoalkane, Epoxide, Glycerole, Haloacetamine, Halotriazine, Hydrazine, Hydroxylamine, Imidoester, Isocyanate, Isothiocyanate, Maleimide, Phosphoramidite, Silylhalogene, Sulfonsäureester und Sulfonsäurechloride.
und die weiteren Reste unabhängig voneinander ausgewählt sind aus der Gruppe H, Alkyl (C₁-C₁₀), Alkoxy (C₁-C₁₀), Trifluormethyl, Halogen, Sulfonsäure, Sulfonat, Phosphorsäure und Phosponat.

2. Fluorochrom nach Anspruch 1,
**dadurch gekennzeichnet, dass** mindestens einer der Reste R₁, R₆ bis R₁₀ und R₁₈ bis R₂₁ eine reaktive Gruppe ist.

3. Fluorochrom nach Anspruch 2,
**dadurch gekennzeichnet, dass** mindestens einer der Reste R₆ bis R₉, und R₁₈ bis R₂₁ eine reaktive Gruppe ist.

4. Fluorochrom nach mindestens einem der vorhergehenden Ansprüche,
**dadurch gekennzeichnet, dass** die reaktive Gruppe ausgewählt ist aus der Gruppe bestehend aus Succinimidylester, Maleiimid, und Phosphoramidit.

5. Fluorochrom nach mindestens einem der vorhergehenden Ansprüche,
**dadurch gekennzeichnet, dass** mindestens einer der Reste R₁ bis R₂₁ hydrophil ist, ausgewählt aus der Gruppe der Carbonsäuren, Kohlenhydrate, Peptide, Nukleinsäuren, Sulfonsäuren, Sulfonate, Thiole, Phosphorsäuren, Phosphonate, Phosphordiester, Phosphortriester, Amine, Halogene, Alkohole, Polyole und Polyether, ist.

6. Fluorochrom nach Anspruch 5,
**dadurch gekennzeichnet, dass** die Carbonsäuren ausgewählt sind aus der Gruppe der mono-, bi- und multifunktionellen aliphatischen Carbonsäuren mit einer Kohlenstoffkette bis zu 10 Atomen, und die Kohlenhydrate ausgewählt aus der Gruppe der Monosaccharide wie Triosen, Tetrosen, Pentosen, Hexosen oder Heptosen, Disaccharide wie Saccharose, Lactose, Trehalose oder Maltose, Oligosaccharide wie Cyclodextrine oder Raffinose und Polysaccharide wie Dextrane oder Chitosane, einschließlich aller 0- und N-glykosidischen Modifikationen und Kombinationen hiervon.

7. Fluorochrom nach mindestens einem der Ansprüche 5 oder 6,
**dadurch gekennzeichnet, dass** mindestens einer der Reste R₁, R₄, R₆ bis R₁₀, R₁₃, R₁₈ bis R₂₁ eine hydrophile Gruppe ist.

8. Fluorochrom nach Anspruch 7,
**dadurch gekennzeichnet, dass** mindestens einer der Reste R₆ bis R₉, und R₁₈ bis R₂₁ eine hydrophile Gruppe ist.

9. Fluorochrom nach mindestens einem der vorhergehenden Ansprüche,
**dadurch gekennzeichnet, dass** das Gegenion W⁻ ausgewählt ist aus der Gruppe bestehend aus Halogenid, Tosylat und Perchlorat.

10. Fluorochrom nach mindestens einem der vorhergehenden Ansprüche,
**dadurch gekennzeichnet, dass** jeweils zwei am aromatischen Ring ortho-ständige Reste unter Ausbildung von mindestens einem aromatischen, carbo- oder heterocyclischen Ring verbrückt sind.

11. Fluorochrom nach mindestens einem der vorhergehenden Ansprüche,
**dadurch gekennzeichnet, dass** das Zielmolekül ein Biomolekül aus der Gruppe der Antikörper, Proteine, Peptide, Enzymsubstrate, Hormone, Lymphokine, Metabolite, Rezeptoren, Antigene, Haptene, Lecitine, Toxine, Kohlenhydrate, Oligosaccharide, Polysaccharide, Nukleinsäuren, Desoxyribonukleinsäuren, derivatisierte Desoxyribonukleinsäuren, derivatisierte Nukleinsäuren, DNA-Fragmente, RNA- Fragmente, Arzneimittel, Viruspartikel, Viruskomponenten, Hefen, Hefekomponenten, Bakterien, Bakterienkomponenten, Blutzellen, Blutzellenkomponenten, biologischen Zellen, nichtzellulären Blutkomponenten, Gifte ist.

12. Fluorochrom nach mindestens einem der vorhergehenden Ansprüche,
**dadurch gekennzeichnet, dass** das Zielmolekül ein nicht biologisches Molekül aus der Gruppe der Polymere, Polymerteilchen, Glasteilchen, Glasoberflächen, Kunststoffoberflächen, Kunststoffteilchen, Polymermembranen, Metalle, elektrischen Leiter und/oder Halbleiter ist.

13. Fluorochrom nach mindestens einem der vorhergehenden Ansprüche,
**dadurch gekennzeichnet dass** das Fluorochrom an die Oberfläche von Nano- oder Micropartikeln auf der Basis organisch chemischer Polymere angebunden oder in diese Partikel eingeschlossen wird, um diese in analytischen Verfahren einzusetzen.

14. Fluorochrom nach mindestens einem der Ansprüche 1 bis 13 **gekennzeichnet durch** eine Verbindung der nachfolgenden Formel II 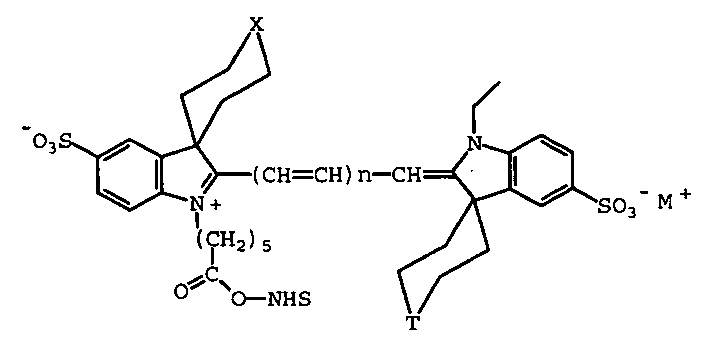 mit M⁺ = Metallkation sowie X, T, W⁻ und n entsprechend Anspruch 1 definiert.

15. Fluorochrom nach mindestens einem der Ansprüche 1 bis 13 **gekennzeichnet durch** eine Verbindung der nachfolgenden Formel III 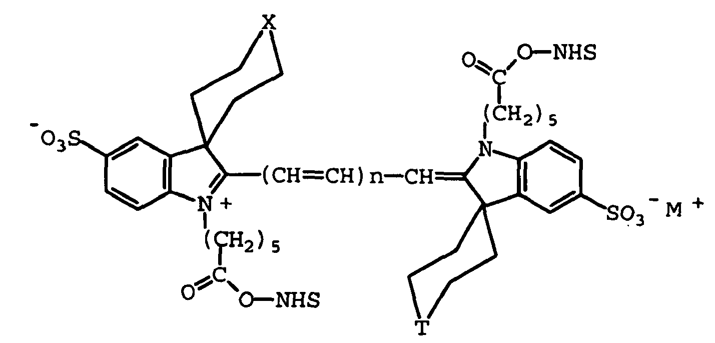 mit M⁺ = Metallkation sowie X, T, W⁻ und n entsprechend Anspruch 1 definiert.

16. Fluorochrom nach mindestens einem der Ansprüche 1 bis 13 **gekennzeichnet durch** eine Verbindung der nachfolgenden Formel IV 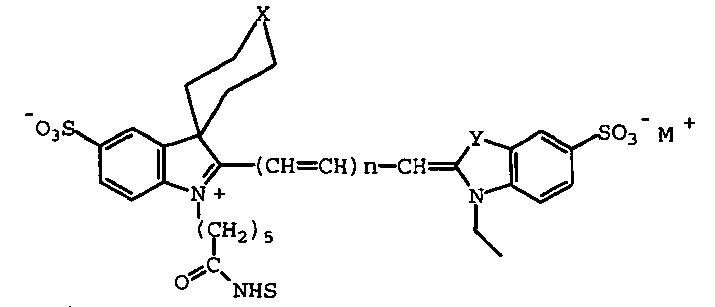 mit M⁺ = Metallkation sowie X, Y, T, W⁻ und n entsprechend Anspruch 1 definiert.

17. Fluorochrom nach mindestens einem der Ansprüche 1 bis 13 **gekennzeichnet durch** eine Verbindung der nachfolgenden Formel V 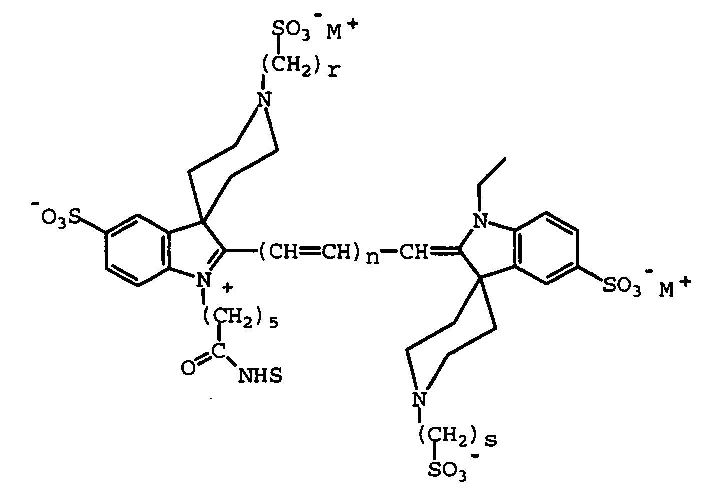 mit M⁺ = Metallkation, W⁻ und n entsprechend Anspruch 1 definiert, NHS = N-Hydroxy-succinimid, r und s unabhängig voneinander = 1-8.

18. Fluorochrom nach mindestens einem der Ansprüche 1 bis 13 **gekennzeichnet durch** eine Verbindung der nachfolgenden Formel VI 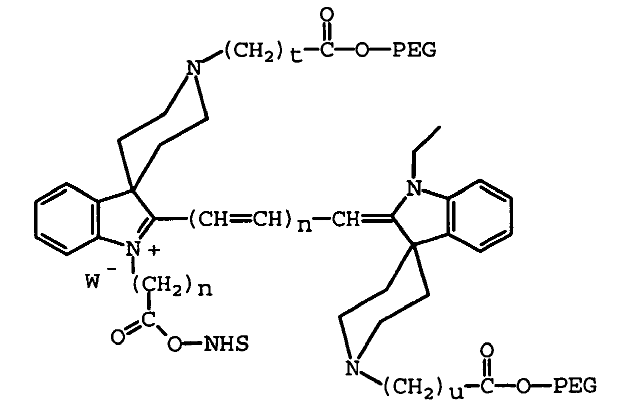 mit W⁻ und n entsprechend Anspruch 1 definiert, sowie NHS = N-Hydroxy-succinimid, PEG = Polyethylenglykol t und u unabhängig voneinander = 1-8.

19. Fluorochrom nach mindestens einem der Ansprüche 1 bis 13 **gekennzeichnet durch** eine Verbindung der nachfolgenden Formel VII 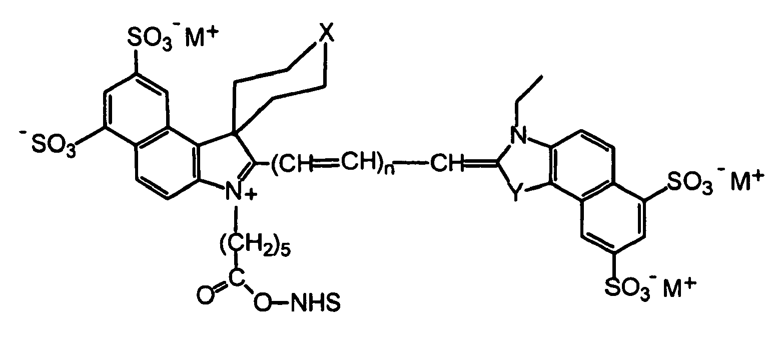 mit M⁺ = Metallkation, X, Y, T, W⁻ und n entsprechend Anspruch 1 definiert

20. Fluorochrom nach mindestens einem der Ansprüche 1 bis 13 **gekennzeichnet durch** eine Verbindung der nachfolgenden Formel VIII 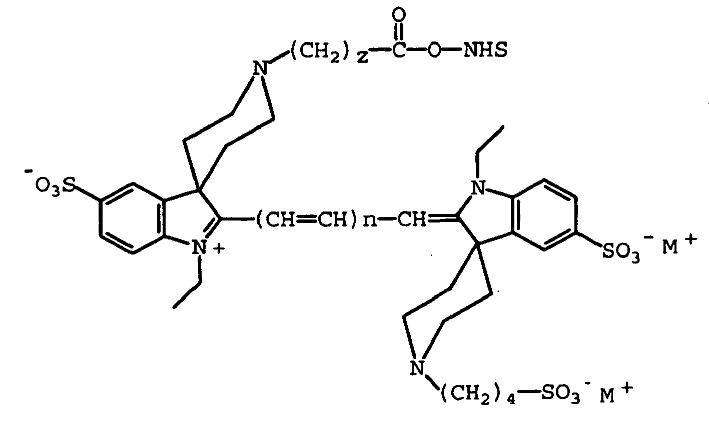 mit M⁺ = Metallkation, X, Y, T, W⁻ und n entsprechend Anspruch 1 definiert, z = 0, 1-6.

21. Verwendung einer Verbindung gemäß Anspruch 1 bis 20 zusammen mit einem anderen Farbstoff oder einem anderen Konjugat als Fluoreszenz Resonanz Energie System

22. Verwendung einer Verbindung gemäß Anspruch 1 bis 20 zur Markierung und/oder Detektion von Nukleinsäuren

23. Verwendung nach Anspruch 22 wobei als Nukleinsäure das Produkt einer Amplifikationsreaktion detektiert wird.

24. Verwendung der Fluorochrome nach mindestens einem der Ansprüche 1 bis 20, als Fluoreszenzfarbstoff zur Kopplung an biologisch oder nicht biologische Zielmoleküle, die in analytischen Verfahren eingesetzt werden.

## Claims

1. A fluorescent dye of the general formula I 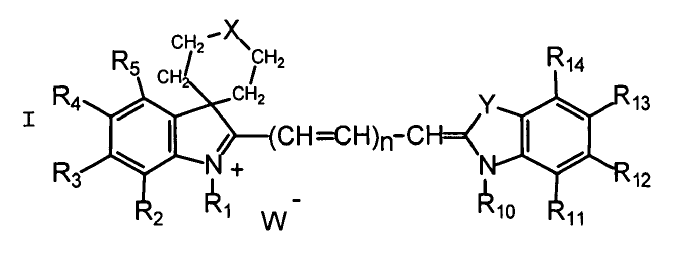 with X = 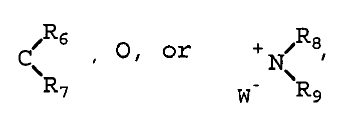 and Y is O, NR₁₇, 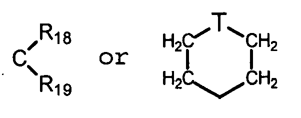 with T is 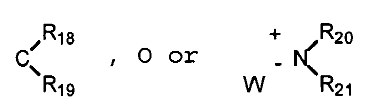 with n = 0, 1, 2, or 3 and
W⁻ = counter ion, preferably halogenide, perchlorate or tosylate,
wherein at least one of the residues R₁ to R₂₁ is selected from
a chemical reactive group for covalent coupling to a target molecule selected from the group of carboxylic acids, activated esters, acylazides, acylhalogenides, acylnitriles, aldehydes, anhydrides, arylamides, alkylhalogenides, anilines, alkylsulfonates, arylhalogenides, thioles, azides, aziridines, borates, carbodiimides, diazoalkanes, epoxides, glyceroles, haloacetamines, halotriazines, hydrazines, hydroxylamines, imidoesters, isocyanates, isothiocyanates, maleimides, phosphoramidites, silylhalogene, sulfonic acid esters and sulfonylclorides,
and the other residues are independently selected from the group of H, alkyl (C₁-C₁₀), alkoxy (C₁-C₁₀), trifluoromethyl, halogen, sulfonic acid, sulfonate, phosphoric acid and phosponate.

2. A fluorescent dye according to claim 1,
**characterized** thereby that at least one of the residues R₁, R₆ to R₁₀, R₁₈ to R₂₁ is a reactive group.

3. A fluorescent dye according to claim 2,
**characterized** thereby that at least one of the residues R₆ to R₉, and R₁₈ to R₂₁ is a reactive group.

4. A fluorescent dye according to at least one of the preceding claims,
**characterized** thereby that the reactive group is selected from the group of succinimidylesters, maleiimides, epoxides, hydrazines and phosphoramidites.

5. A fluorescent dye according to at least one of the preceding claims,
**characterized** thereby that at least one of the residues R₁ to R₂₁ is hydrophilic, selected from the group of carboxylic acids, carbohydrates, peptides, nucleic acids, sulfonic acids, sulfonates, thioles, phosporic acids, phosphonates, phosphordiester, phoshortriester, amines, halogenes, alcoholes, polyoles or polyether.

6. A fluorescent dye according to claim 5,
**characterized** thereby that the carboxylic acid is selected from the group of mono-, bi- and multifunctional aliphatic carboxylic acids with a carbon chain of up to 10 atoms and the carbohydrates selected from the group of monosaccharides, such as trioses, tetroses, pentoses, hexoses or heptoses, disaccharides like sucrose, lactose, trehalose or maltose, oligosaccharides like cyclodextrine or raffinose and polysaccharides like dextran or chitosane, including all 0- and N-glycosidic modifications and combinations thereof.

7. A fluorescent dye according to at least one of the claims 5 or 6,
**characterized** thereby that at least one of the residues R₁, R₄, R₆ to R₁₀, R₁₃, and R₁₈ to R₂₁ is a hydrophilic group.

8. A fluorescent dye according to claim 7,
**characterized** thereby that at least one of the residues R₆ to R₉, and R₁₈ to R₂₁ is a hydrophilic group.

9. A fluorescent dye according to at least one of the preceding claims,
**characterized** thereby that in which the counter ion W⁻ is selected from the group of halogenide, tosylate and perchlorate.

10. A fluorescent dye, according to at least one of the preceding claims
**characterized** thereby that each pair of aromatic ortho-substituted residues can be combined to form at least one adjacent carbo- or heterocyclic aromatic ring.

11. A fluorescent dye according to at least one of the preceding claims,
**characterized** thereby that the target molecule is a biomolecule selected from the group of antibodies, proteines, peptides, enzyme substrates, hormones lymphokines, metabolites, receptors, antigenes, haptenes, lectines, toxines, carbohydrates, oligosaccharides, polysaccharides, nucleic acids, desoxy ribonucleic acids (DNA), derivatized DNA, derivatized nucleic acids, DNA-fragments, RNA- fragments, drugs, virus particles, virus components, yeast, yeast components, bacteria, bacterial components, blood cells, components of blood cells, biological cells, non cellular blood components and toxines

12. A fluorescent dye according to at least one of the preceding claims,
**characterized** thereby that the target molecule is a non biological molecule selected from the group of polymeres, polymer particles, glas particles, glas surfaces, plastic surfaces, plastic particles, polymer membranes, metals, electrical conductors and/or electrical semiconductors.

13. A fluorescent dye according to at least one of the preceding claims,
**characterized** thereby that the fluorescent dye is bound onto the surface or is incorporated into nano- or microparticles of the basis of organic polymers and the use of said particles for analytical applications (methods).

14. A fluorescent dye according to at least one of the claims 1-13, **characterized by** a compound with the following formula II 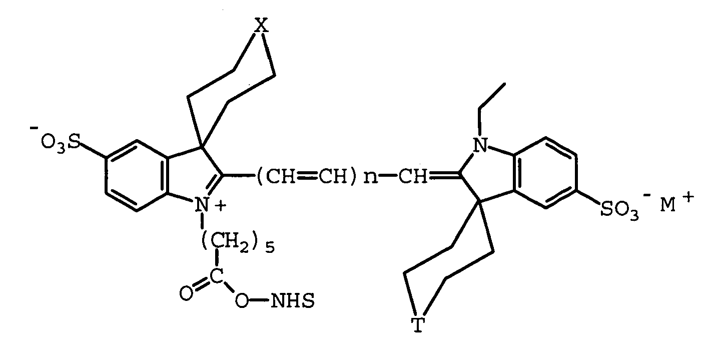 with M⁺ = a metal cation, X, T, W⁻ and n are defined according to claim 1.

15. A fluorescent dye according to at least one of the claims 1-13, **characterized by** a compound with the following formula III 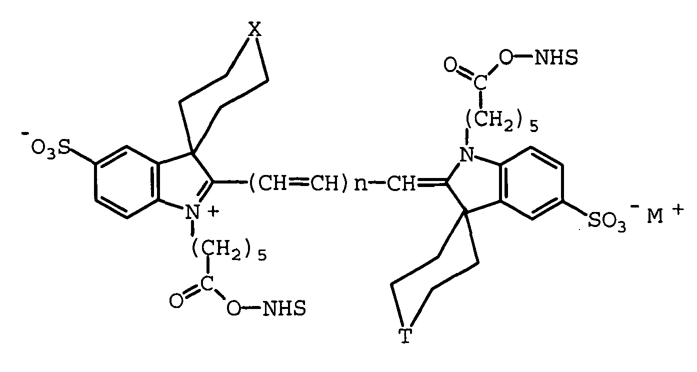 with M⁺ = a metal cation, X, T, W⁻ and n are defined according to claim 1.

16. A fluorescent dye according to at least one of the claims 1-13, **characterized by** a compound with the following formula IV 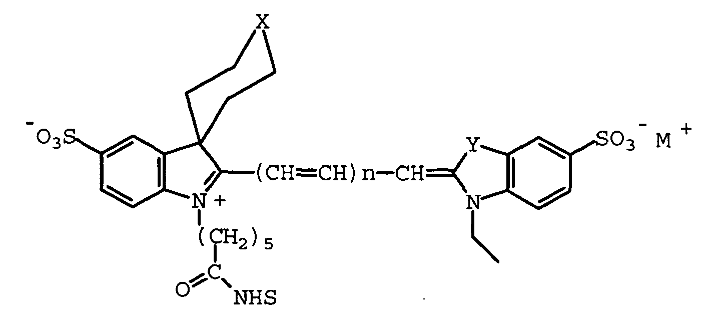 with M⁺ = a metal cation, X, Y, T, W⁻, m and n are defined according to claim 1.

17. A fluorescent dye according to at least one of the claims 1-13, **characterized by** a compound with the following formula V 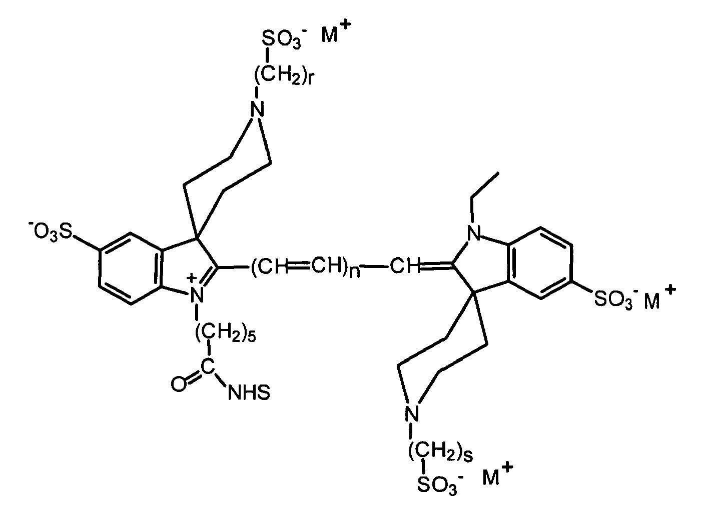 with M⁺ = metal cation, W⁻ and n defined according to claim 1, NHS = N-hydroxy-succinimide, r and s independently from each other = 1 to 8.

18. A fluorescent dye according to at least one of the claims 1-13, **characterized by** a compound with the following formula VI 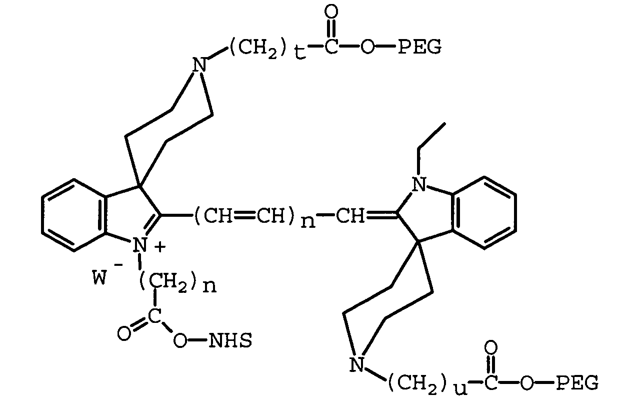 with W⁻ and n defined according to claim 1, NHS = N-hydroxy-succinimid, PEG = poly ethylen glycole and with t and u independently from each other = 1 to 8.

19. A fluorescent dye according to at least one of the claims 1-13, **characterized by** a compound with the following formula VII 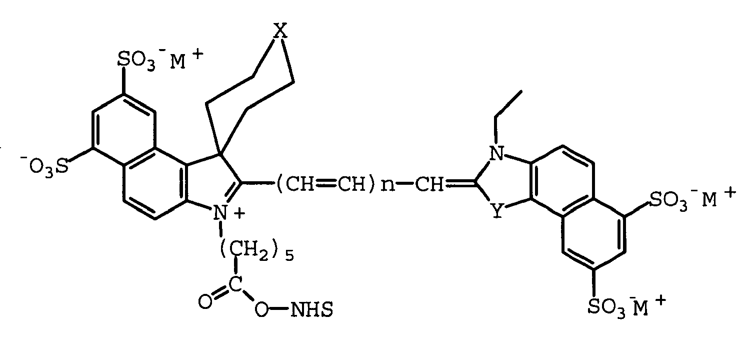 with M⁺ = metal cation, X, Y, T, W⁻ and n defined according to claim 1.

20. A fluorescent dye according to at least one of the claims 1-13, **characterized by** a compound with the following formula VIII 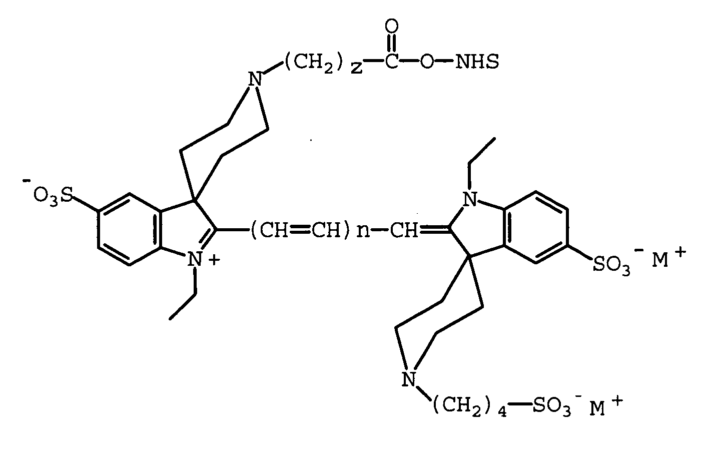 with M⁺ = metal cation, X, Y, T, W⁻ and n defined according to claim 1, z =0, 1 to 6.

21. Use of a compound according to claim 1 to 20 together with another fluorescent dye or another conjugate in a fluorescence resonance energy transfer system.

22. Use of a compound according to claim 1 to 20 for labelling and/or detection of nucleic acids.

23. Use according to claim 22, where the nucleic acid is detected as product of an amplification reaction.

24. Use of a compound according to at least one of the claims 1 to 20 as fluorescent dye for coupling to biological and non-biological target molecules, which are used in analytical methods.

## Revendications

1. Une molécule fluorescente de formule général I 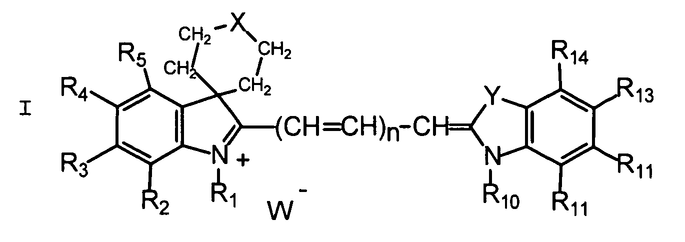 où X = 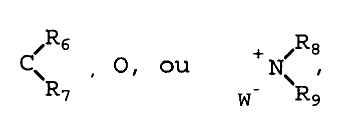 et Y est O, NR₁₇, 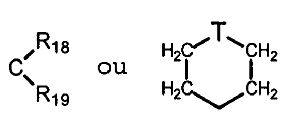 où T est 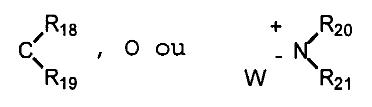 où n = 0, 1, 2, ou 3 et
W⁻ = contre ion, sélectionné préférablement dans les halogénures, perchlorate ou tosylate,
dans laquelle au moins un des résidues de R₁ à R₂₁ est choisi comme
une fonction chimique réactive pour des couplages covalents avec une molécule cible qui est choisie parmi les acides carboxyliques, les esters activés, les acylazides, les halogénures d'acyles, les acylnitriles, les aldéhydes, les anhydrides, les arylamides, les halogénures d'alkyles, les anilines, les alkylsulfonates, les halogénures d'aryles, les thiols, les azides, les aziridines, les borates, les carbodiimides, les diazoalkanes, les époxides, les glycéroles, les haloacétamines, les halotriazines, les hydrazines, les hydroxylamines, les imidoesters, les isocyanates, les isothiocyanates, les maléimides, les phosphoramidites, les silylhalogènes, les esters d'acides sulfoniques et les chlorures de sulfonyles,
et l'autre résidu est sélectionné indépendamment dans le groupe suivant: H, alkyl (C₁-C₁₀), alkoxy (C₁-C₁₀), trifluorométhyl, halogène, acide sulfonique, sulfonate, acide phosphorique et phosponate.

2. une molécule fluorescente selon la revendication 1,
**caractérisée** de façon à ce que au moins un des résidus R₁, de R₆ à R₁₀, de R₁₈ à R₂₁ soit un groupe réactif.

3. une molécule fluorescente selon la revendication 2,
**caractérisée** de façon à ce que au moins un des résidus de R₆ à R₉, de R₁₈ à R₂₁ soit un groupe réactif.

4. une molécule fluorescente selon au moins une des revendications précédentes,
**caractérisée** de façon à ce que le résidu réactif soit choisi dans le groupes des succinimidylesters, des maléiimides, des époxides, des hydrazines et des phosphoramidites.

5. une molécule fluorescente selon au moins une des revendications précédentes,
**caractérisée** de façon à ce que au moins un des résidus de R₁ à R₂₁ soit hydrophile, sélectionné dans le groupes des acides carboxyliques, des carbohydrates, des peptides, des acides nucléiques,des acides sulfoniques, des sulfonates, des thiols, des acides phosporiques, des phosphonates, des phosphodiester, des phoshotriester, des amines, des halogènes, des alcools, des polyoles ou des polyéther.

6. Une molécule fluorescente selon la revendication 5,
**caractérisée** de façon à ce que l'acide carboxylique soit sélectionné dans le groupe des acides carboxyliques aliphatiques mono- bi- et multifonctionels dont la chaîne carbonnée comprend jusqu'à 10 atomes et dont le carbohydrate est sélectionné dans le groupe des monosaccharides, comme les trioses, tétroses, pentoses, hexoses ou heptoses, des disaccharides comme le saccharose, le lactose, le tréhalose ou le maltose, des oligosaccharides comme les cyclodextrines ou les raffinoses, et des polysaccharides comme les dextranes ou chitosanes, incluant toutes les modifications O- et N- glycosidiques et autres combinaisons.

7. Une molécule fluorescente selon au moins une des revendications 5 ou 6,
**caractérisée** de façon à ce que au moins des résidues R₁, R₄, de R₆ à R₁₀, R₁₃, et de R₁₈ à R₂₁ soit un groupe hydrophile.

8. Une molécule fluorescente selon la revendication 7,
**caractérisée** de façon à ce que au moins un des résidues R₆ à R₉, et de R₁₈ à R₂₁ soit un groupe hydrophile.

9. Une molécule fluorescente selon au moins une des revendication précédentes,
**caractérisée** de façon à ce que le contre ion W⁻ soit choisi dans le groupe des halogénures, tosylate ou perchlorate.

10. Une molécule fluorescente, selon au moins une des revendication précédentes
**caractérisée** de façon à ce que chaque paire de residues aromatiques orthosubstitués puisse être combinée pour former au moins un noyau aromatique carbo- ou hétérocyclique adjacent.

11. Une molécule fluorescente, selon au moins une des revendications précédentes
**caractérisée** de façon à ce que la molécule cible soit une biomolécule sélectionnée dans le groupe des anticorps, des protéines, des peptides, des substrats d'enzymes, des hormones lymphokines, des métabolites, des récepteurs, des antigènes,des haptènes, des léctines, des toxines, des carbohydrates, des oligosaccharides, des polysaccharides, des acides nucléiques, des acides désoxy ribonucléique (ADN), ADN dérivatisés, des acides nucléiques dérivatisés, des fragments d'ADN, des fragments d'ARN, des médicaments, des particules virales, des composants de virus, des levures, des composants de levures, des bactéries, des composants bactériens, des cellules sanguines, des cellules biologiques, des composants cellulaires non sanguin et des toxines

12. Une molécule fluorescente, selon au moins une des revendication précédentes
**caractérisée** de façon à ce que la molécule cible soit une molécule non biologique sélectionnée dans le groupe des polymers, des particules polymériques, des particules vitreuses, des surfaces vitreuses, des surfaces plastiques, des particules plastiques, des membranes polymères, des métaux, des conducteurs électriques et/ou semiconducteurs électriques.

13. Une molécule fluorescente, selon au moins une des revendication précédentes
**caractérisée** de façon à ce que la molécule fluorescente aie un lien sur la surface ou soit incorporée dans des nano- ou microparticules de polymères organiques usuels et dont la dite particule est utilisée pour des applications analytiques.

14. Une molécule fluorescente, selon au moins une des revendications 1 à 13, **caractérisée par** le composé de formule II suivante 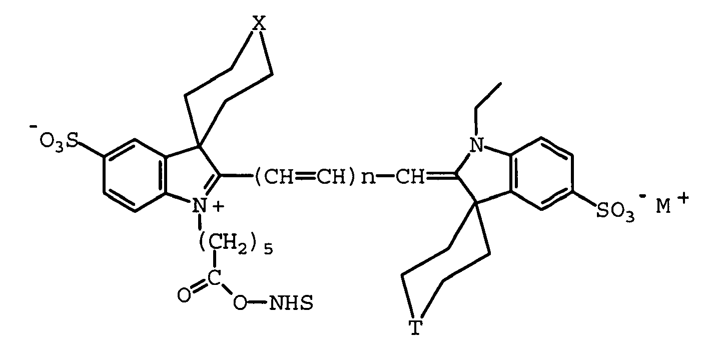 où M⁺ = cation métallique, X, T, W⁻ et n sont définis selon la revendication 1.

15. Une molécule fluorescente, selon au moins une des revendications 1 à 13, **caractérisée par** le composé de formule III suivante 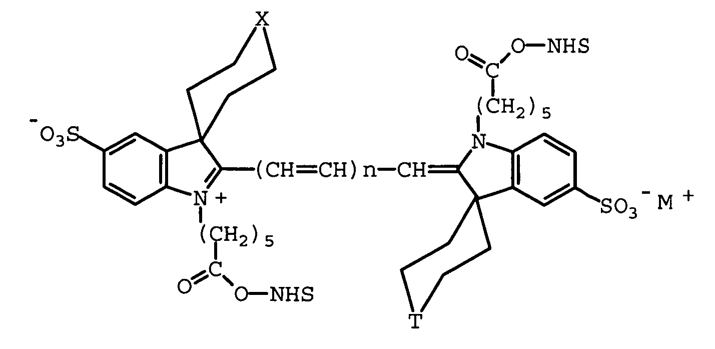 où M⁺ = cation métallique, X, T, W⁻ et n sont définis selon la revendication 1.

16. Une molécule fluorescente, selon au moins une des revendications 1 à 13, **caractérisée par** le composé de formule IV suivante 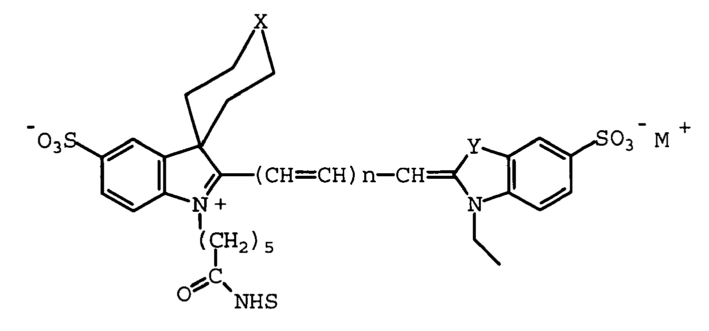 où M⁺ = cation métallique, X, T, W⁻ et n sont définis selon la revendication 1.

17. Une molécule fluorescente, selon au moins une des revendications 1 à 13, **caractérisée par** le composé de formule V suivante 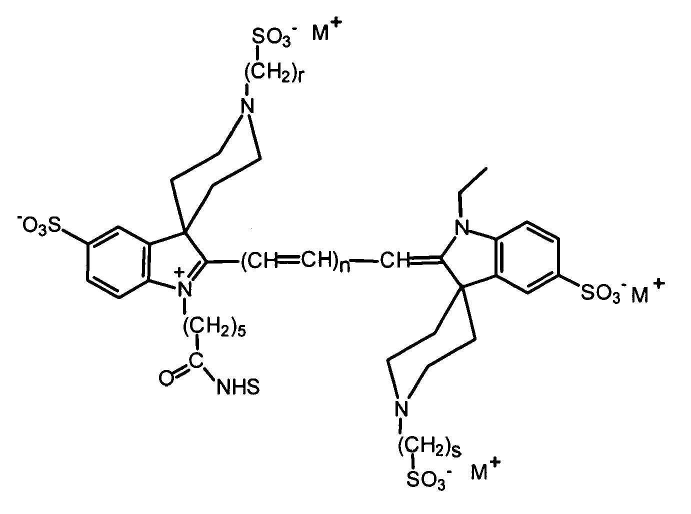 où M⁺ = cation métallique et n défini selon la revendication 1. NHS = N-hydroxy-succinimide, r et s sont indépendants et choisis parmi les entiers de 1 = 8.

18. Une molécule fluorescente, selon au moins une des revendications 1 à 13, **caractérisée par** le composé de formule VI suivante 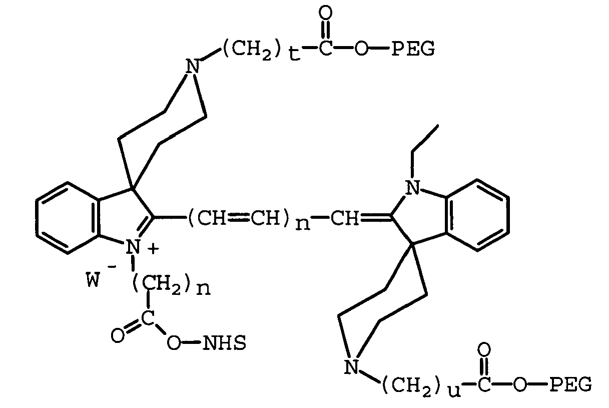 où W⁻ et n sont définis selon la revendication 1, NHS = N-hydroxy-succinimide, PEG = poly éthylèn glycol, t et u sont indépendants et choisis parmi les entiers de 1 à 8

19. Une molécule fluorescente, selon au moins une des revendications 1 à 13, **caractérisée par** le composé de formule VII suivante 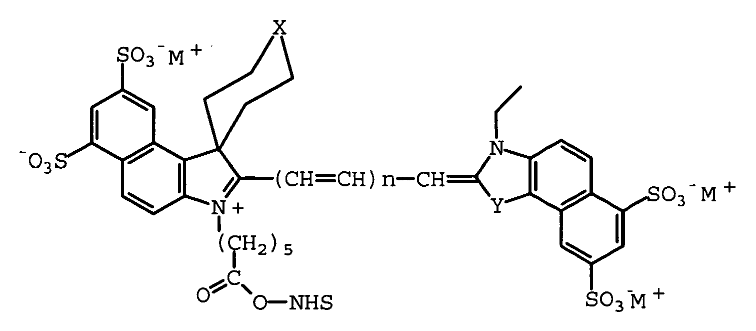 où M⁺ = cation métallique, X, Y, T, W⁻ et n sont définis selon la revendication 1.

20. Une molécule fluorescente, selon au moins une des revendications 1 à 13, **caractérisée par** le composé de formule VIII suivante 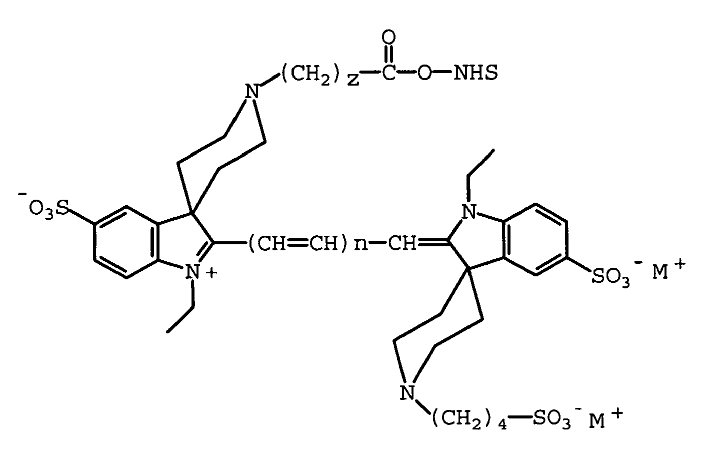 où M⁺ = cation métallique, X, Y, T, W⁻ et n sont définis selon la revendication 1 et z choisi parmi les entiers de 0 à 6.

21. Utilisation d'un composé selon les revendications de 1 à 20 communément avec une autre molécule fluorescente ou un autre conjugué dans un système de transfert d'énergie résonante fluorescente.

22. Utilisation d'un composé selon les revendications de 1 à 20 est utilisée pour marquer et/ou détecter des acides nucléiques.

23. Utilisation selon la revendication 22, où l'acide nucléique est détecté comme le produit d'une réaction d'amplification.

24. Utilisation d'un composé selon les revendications de 1 à 20 comme molécule fluorescente dans des couplages avec des molécules cibles biologiques ou non biologiques, qui sont utilisées dans des méthodes analytiques.
